# EUROPEAN PATENT APPLICATION

(11) **EP 4 603 577 A1**
(43) Date of publication of application: **20.08.2025**
(21) Application number: 23877589.4
(22) Date of filing: 05.10.2023
(51) Int. Cl.: C12N 5/0775, A61K 35/545, A61K 35/28, A61K 38/18, A61P 9/00, A61P 25/00, A61L 27/38, A61L 27/50

(54) **NOVEL ANGIOGENIC STEM CELL**

(30) Priority: 12.10.2022 KR 20220130573; 28.07.2023 KR 20230099140
(71) Applicant: Elphis Cell Therapeutics, Seoul 02455 (KR)
(72) Inventor: YIM, Sung Vin, Seoul 02095 (KR); HONG, Hyun Sook, Seoul 05619 (KR); HWANG, Dae Yeon, Goyang-si Gyeonggi-do 10450 (KR); SON, Young Sook, Seoul 06305 (KR); PARK, Ki Sook, Seoul 06374 (KR); PARK, Ga Bee, Asan-si Chungcheongnam-do 31533 (KR); HONG, Seon Min, Yongin-si Gyeonggi-do 16846 (KR); KIM, Dae Wook, Yongin-si Gyeonggi-do 17066 (KR)
(74) Representative: Isarpatent
(86) International application number: PCT/KR2023/015279
(87) International publication number: WO 2024/080661

(57) **Abstract**

The present disclosure relates to vMSC, which is a stem cell that is newly identified and has angiogenic properties. vMSCs cultured under specific culture conditions and identified, according to the present disclosure, are novel MSCs that express a CD141 cell surface antigen, unlike conventional MSCs, and the vMSC has excellent cell expansion ability, has a direct angiogenic effect, and has an autocrine effect unlike conventional stem cells, as well as a paracrine effect by other MSCs. Thus, the vMSCs have a higher survival and retention rate in tissues than conventional stem cell therapeutic agents, and can be used as a stem cell therapeutic agent for blood vessel regeneration that directly forms blood vessels.

## Description

### [Technical Field]

The present disclosure relates to vMSCs, pluripotent stem cells that are newly identified and have angiogenic properties.

### [Background Art]

New blood vessels are generated by angiogenesis, arteriogenesis and vasculogenesis, and the angiogenesis is related to the proliferation and migration of endothelial cells that grow from already existing mature endothelial cells. The arteriogenesis is a process of remodeling existing arteriolar connections into collateral vessels, and the vasculogenesis proceeds through a process in which endothelial progenitor cells differentiate into mature endothelial cells. Therefore, the endothelial progenitor cells circulating from the bone marrow migrate to a vascular damage area, and are involved in formation of new blood vessels through direct insertion into newly formed blood vessels or secretion of various angiogenic and trophic factors. Therefore, the endothelial progenitor cells are attracting attention as a potential target in the field of regenerative medicine and therapeutic purposes through revascularization. Accordingly, studies have been conducted to increase the functions of the endothelial progenitor cells, and for example, studies on the production of recombinant EPCs obtained by modifying ex vivo genes have been conducted, and a technique for increasing the proangiogenic capacity of EPCs using a vascular endothelial growth factor (VEGF) or hypoxiainducible factor-1α has been studied. Currently, cells used for cell therapy in ischemic vascular disease are largely divided into two types. The cells include endothelial progenitor cells (EPCs) that play a role in blood vessel regeneration and mesenchymal stem cells that secrete large amounts of growth factors that contribute to angiogenesis to have a paracrine effect that indirectly helps angiogenesis. However, existing EPCs are a cell group in which in vitro cell expansion is very difficult, and it is also difficult to maintain functions during cell expansion, and there is a high risk of problems such as immune rejection in the case of allogeneic and xenogeneic cells, and have a limitation that it is difficult to differentiate and function into blood vessels with complex structures. In addition, conventional therapeutic agents developed based on mesenchymal stem cells targeting ischemic brain neurological disease are lost without engraftment and thus do not exhibit a clear therapeutic effect in clinical trials by depending on the paracrine effect rather than angiogenesis-based treatment.

Cell therapy products, including stem cell therapy products, are defined as medicines used for treatment, diagnosis, and prevention through a series of actions, such as proliferating or selecting living autologous, allogeneic, or xenogeneic cells *in vitro* or changing the biological characteristics of cells through other methods to restore the functions of cells and tissues. Cell therapy transplantation using cell therapy products is used for treatment by injecting an implant to a subject to be received or creating human tissues with these cells by performing a series of actions of separating and preparing each cell material composed of a plurality of cells capable of replacing various tissues that constitute the body and have been expressed as tissues similar to the normal or healthy state in the body, and changing the biological characteristics of the cells through a culture process in an in vitro environment. The stem cell therapy product refers to a cell therapy product that specifically uses stem cells, and currently, research has been actively conducted in representative application fields where recovery and regeneration of lost cells are essential, but not well achieved, such as neurological diseases, heart diseases, lung diseases, liver diseases, and cancers. Stem cells have great potential in cell therapy because the stem cells have the multiple differentiating potential to induce differentiation into the cells required in damaged tissues. However, at the current level of development, the survival rate after transplantation into the body is not high, so that actually, it is difficult to find examples of widespread and stable success in clinical application.

### [Disclosure]

### [Technical Problem]

An object of the present disclosure is to provide novel stem cells.

Another object of the present disclosure is to provide a cell therapeutic agent composition for blood vessel regeneration or angiogenesis.

Yet another object of the present disclosure is to provide a graft material for blood vessel regeneration.

Still another object of the present disclosure is to provide a pharmaceutical composition for preventing or treating vascular disease or vascular dysfunction.

Still another object of the present disclosure is to provide a pharmaceutical composition for preventing or treating brain neurological disease.

Still another object of the present disclosure is to provide a method for preparing novel stem cells.

Still another object of the present disclosure is to provide a use of stem cells expressing a CD141 cell surface antigen for blood vessel regeneration or angiogenesis.

Still another object of the present disclosure is to provide a method for blood vessel regeneration or angiogenesis.

Still another object of the present disclosure is to provide a method for treating vascular disease or vascular dysfunction.

Still another object of the present disclosure is to provide a method for treating brain neurological disease.

### [Technical Solution]

To achieve the object, an aspect of the present disclosure provides stem cells expressing a CD141 cell surface antigen.

Another aspect of the present disclosure provides a therapeutic agent composition for blood vessel regeneration or angiogenesis including the stem cells.

Yet another aspect of the present disclosure provides a graft material for blood vessel regeneration.

Still another aspect of the present invention provides a pharmaceutical composition for preventing or treating vascular disease or vascular dysfunction.

Still another aspect of the present disclosure provides a pharmaceutical composition for preventing or treating brain neurological disease.

Still another aspect of the present disclosure provides a method for preparing stem cells expressing a CD141 cell surface antigen.

Still another object of the present disclosure provides a use of stem cells expressing a CD141 cell surface antigen for blood vessel regeneration or angiogenesis.

Still another aspect of the present disclosure provides a method for blood vessel regeneration or angiogenesis.

Still another aspect of the present disclosure provides a method for treating vascular disease or vascular dysfunction.

Still another aspect of the present disclosure provides a method for treating brain neurological disease.

### [Advantageous Effects]

According to the present disclosure, multipotent stem cells (vMSCs) cultured and obtained under specific culture conditions share several characteristics with conventional mesenchymal stem cells or mesenchymal stromal cells (MSCs), but are novel MSCs that express a CD141 cell surface antigen, unlike conventional MSCs. The vMSC has excellent cell expansion ability, has a direct angiogenic effect, and has an autocrine effect of blood vessel regeneration-related factors unlike conventional stem cells, as well as a paracrine effect of the blood vessel regeneration-related factors by other MSCs. Thus, the vMSCs have a higher survival and retention rate in tissues than conventional stem cell therapeutic agents, and can be used as a stem cell therapeutic agent for blood vessel regeneration that directly forms blood vessels.

### [Description of Drawings]

FIG. 1 is a diagram showing colony shapes of CD141⁺vMSCs formed in various extracellular matrices.
FIG. 2 is a diagram of confirming the cell characteristics according to expression of CD141⁺vMSC markers.
FIG. 3 is a diagram showing results of discovering novel markers specific to CD141⁺vMSCs that are distinguished from BM-MSCs, through marker screening.
FIG. 4 is a diagram of evaluating the cell expansion ability of CD141⁺vMSC.
FIG. 5 is a diagram of evaluating multipotency which is a stem cell characteristic of CD141⁺vMSC.
FIG. 6 is a diagram of confirming the vascular network formation ability of CD141⁺vMSC, BM-MSC, and HUVEC according to a culture medium (scale bar = 200 µm).
FIG. 7 is a diagram of analyzing the measurement of autocrine HGF expression levels of CD141⁺vMSC and signaling activity through c-MET receptors:
FIG. 7A: HGF concentration in a culture medium when CD141⁺vMSC was cultured in an EGM-2-FBS+2%hPL medium;
FIG. 7B: HGF concentration in a culture medium when BM-MSC was cultured in a StemMACS medium;
FIG. 7C: HGF concentration in a culture medium when CD141⁺vMSCs and BM-MSCs at Passage 3 were cultured under the same conditions in an angiogenic growth factor-deficient medium (MEM alpha + 0.2% hPL);
FIG. 7D: Expression level and c-Met activity of HGF receptor (c-Met) (phosphorylation) by passage of CD141⁺vMSC and BM-MSC;
FIG. 7E: Quantification of expression level of C-Met; and
FIG. 7F: Quantification of activity (phosphorylation) of C-Met.
FIG. 8 is a diagram of analyzing the vascular sprouting ability by HGF in CD141⁺vMSC spheroids and BM-MSC spheroids:
FIG. 8A: CD141⁺vMSCs; and
FIG. 8B: BM-MSCs.
FIG. 9 is a diagram of analyzing the vascular sprouting ability of CD141⁺vMSC spheroids by bFGF secreted from BM-MSCs.
FIG. 10 is a diagram of confirming the vascular sprouting ability of CD141⁺vMSCs spheroids by VEGF or bFGF.
FIGS. 10A and 10C: Vascular sprouting ability of vMSC spheroids by VEGF or bFGF;
FIGS. 10B and 10D: Vascular sprouting ability of BM-MSC spheroids by VEGF or bFGF; and
FIG. 10E: mRNA expression of VEGFR2 in vMSCs and BM-MSCs (control: HUVECs).
FIG. 11 is a diagram of confirming the promotion of vascular sprouting ability of HUVEC spheroids by HGF secreted from vMSC.
FIG. 12 is a diagram showing results of comparing the characteristics of EPCs and CD141⁺vMSC previously reported in the literature.
FIG. 13 is a diagram of comparing the vascular network formation ability of CD141⁺vMSC and BM-MSC in a normal environment and confirming a cell composition ratio of composite stem cells combined with CD141⁺vMSC and BM-MSC to maximize blood vessel regeneration ability (scale bar = 500 µm).
FIG. 14 is a diagram of confirming compositions of composite stem cells combined with CD141⁺vMSC and BM-MSC to maximize the blood vessel regeneration ability of CD141+vMSC in an inflammatory environment (scale bar = 500 µm).
FIG. 15 is a schematic diagram showing a process of isolating CD141⁺vMSCs from an adipose tissue.
FIG. 16 is a diagram of comparing the cell shapes of CD141⁺vMSC and AD-MSC by passage.
FIG. 17 is a diagram of comparing the cumulative cell numbers of CD141⁺vMSC and AD-MSC.
FIG. 18 is a diagram of comparing cell surface markers of CD141⁺vMSC and AD-MSC.
FIG. 19 is a diagram of comparing the angiogenic ability of CD141⁺vMSC and AD-MSC.
FIG. 20 is a diagram showing the establishment of a critical limb ischemia animal model.
FIG. 21 is a diagram of evaluating the limb salvage efficacy in a critical limb ischemia model by treatment with a composite stem cell combined with CD141⁺vMSC and BM-MSC.
FIG. 22 is a diagram of evaluating ischemic necrosis grades in a critical limb ischemia model by treatment with a composite stem cell combined with CD141⁺vMSC and BM-MSC.
FIG. 23 is a diagram of analyzing changes in blood flow in a critical limb ischemia model by treatment with a composite stem cell combined with CD141⁺vMSC and BM-MSC.
FIG. 24 is a diagram of evaluating limb salvage efficacy to determine a minimal effective dose as a stem cell therapeutic agent of a composite stem cell combined with CD141⁺vMSC and BM-MSC.
FIG. 25 is a diagram of evaluating ischemic necrosis grades and blood flows to determine a minimal effective dose as a stem cell therapeutic agent of a composite stem cell combined with CD141⁺vMSC and BM-MSC.
FIG. 26 is a diagram of evaluating an effect of a composite stem cell combined with CD141⁺vMSC and BM-MSC compared to single administration of each cell as limb salvage efficacy.
FIG. 27 is a diagram of evaluating an effect of a composite stem cell combined with CD141⁺vMSC and BM-MSC compared to single administration of each cell as ischemic necrosis grades.
FIG. 28 is a diagram of evaluating an effect of a composite stem cell combined with CD141⁺vMSC and BM-MSC compared to single administration of each cell as blood flows.
FIG. 29 is a diagram of visually confirming angiogenesis of composite stem cells combined with CD141⁺vMSC and BM-MSC in a critical limb ischemia model:
   White dotted line: an area where blood vessels have been removed; and
   Yellow arrows: newly formed blood vessels.
FIG. 30 is a diagram of confirming angiogenesis of composite stem cells combined with CD141⁺vMSC and BM-MSC in a critical limb ischemia model by immunofluorescence staining:
   White Box: Area where blood vessels have been removed (ischemic location).
FIG. 31 is a diagram of confirming in vivo angiogenesis of composite stem cells combined with CD141⁺vMSC and BM-MSC in a critical limb ischemia model by immunohistochemical staining:
   FIG. 31A: Vessel structure and each marker; and
   FIG. 31B: Immunohistochemical staining result.
FIG. 32 is a diagram of classifying and analyzing in vivo angiogenesis of composite stem cells combined with CD141⁺vMSC and BM-MSC in a critical limb ischemia model according to a vessel diameter:
FIG. 33 is a diagram of confirming the safety of composite stem cells combined with CD141⁺vMSC and BM-MSC.

### [Best Mode]

Hereinafter, embodiments of the present disclosure will be described in detail with reference to the accompanying drawings. However, the following embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used herein are terminologies used to properly express preferred exemplary embodiments of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be understood to imply the inclusion of stated elements, not the exclusion of any other elements.

All technical terms used in the present disclosure, unless otherwise defined, are used as the meaning as commonly understood by those skilled in the related art of the present disclosure. In addition, although preferred methods and samples are described herein, similar or equivalent methods and samples thereto are also included in the scope of the present disclosure. The contents of all publications disclosed as references in this specification are incorporated in the present disclosure.

Throughout this specification, %' used to indicate the concentration of a specific material is solid/solid (w/w)%, solid/liquid (w/v)%, and liquid/liquid (v/v)%, unless otherwise stated.

In one aspect, the present disclosure relates to stem cells expressing a CD141 (thrombomodulin TM) cell surface antigen.

In one embodiment, the stem cells expressing the CD141 cell surface antigen may be vasculogenic multipotent stem cells (vMSCs).

In one embodiment, the cells may be CD141⁺vMSCs (Vasculogenic Multipotent Stem Cells) deposited under accession number KCLRF-BP-00524.

In one embodiment, the vMSCs may be umbilical cord blood-derived, umbilical cord-derived, adipose-derived, or bone marrow-derived, and more preferably bone marrow or adipose-derived.

In one embodiment, the vMSCs may have blood vessel regeneration ability or angiogenic ability.

In one embodiment, the vMSCs may have multipotency and have differentiation potency into adipocytes, osteocytes, chondrocytes, or myocytes.

In one embodiment, the vMSCs may overexpress a hepatocyte growth factor (HGF) and a receptor thereof compared to other stem cells.

In one embodiment, the cells may have a population doubling time (PDT) of 0.5 to 1.5 days.

In one embodiment, the vMSCs may differentiate into endothelial cells in vivo, and the endothelial cells may be CD31 positive.

In one embodiment, the vMSCs may promote angiogenesis by a basic fibroblast growth factor (bFGF) or HGF.

In one embodiment, the vMSCs may express CD105, CD90, CD29, CD73, or CD44 without expressing a CD31, CD309, CD34, eNOS, VE-cadherin, or VEGF receptor.

In one embodiment, the vMSCs may not form new blood vessels by VEGF.

In one embodiment, the stem cells may be adult stem cells, or may be mesenchymal stem cells (MSCs) or have properties thereof.

In one embodiment, the mesenchymal stem cells may be umbilical cord blood-derived mesenchymal stem cells (UCB-MSCs), umbilical cord-derived mesenchymal stem cells (UC-MSCs), adipose-derived mesenchymal stem cells (AD-MSCs) or bone marrow-derived mesenchymal stem cells (BM-MSCs).

The term "stem cell" used herein refers to undifferentiated cells that may differentiate into various cells that constitute biological tissues and regenerate without limitation to form specialized cells of tissues and organs. The stem cells are pluripotent or multipotent cells capable of development. The stem cells proliferate into mature and complete cells of the tissue.

The term "mesenchymal stem cell" used herein refers to an undifferentiated cell having multipotency derived from an adult cell of a mammal including a human, preferably a human, and means a stem cell having multipotency capable of differentiating into adipocytes, bone cells, chondrocytes, muscle cells, nerve cells, and cardiomyocytes. The mesenchymal stem cells may be derived from various adult cells, including, for example, bone marrow, blood, brain, skin, fat (i.e., adipose tissue or adipocytes), umbilical cord blood, and umbilical Wharton's jelly.

The term "differentiation" used herein refers to a phenomenon in which less specialized cells develop into specific cells, and the size, shape, membrane potential, metabolic activity, and response to signals of the cells change into specific types of cells, and a state in which a qualitative difference occurs between parts of a certain biological system that were initially almost homogeneous, or as a result, the system is divided into qualitatively distinguishable subsystems. In particular, differentiation of the stem cells refers to a phenomenon in which stem cells develop in a direction into cells with a specific function.

The term "expression" used herein generally refers to a cellular process by which a biologically active polypeptide is produced from a DNA sequence and exhibits biological activity in a cell. In that meaning, gene expression includes not only transcription and translation processes, but also post-transcriptional and post-translational processes that may affect the biological activity of a gene or gene product. The processes include RNA synthesis, processing and transport, as well as polypeptide synthesis, transport and post-translational modifications, but are not limited thereto.

The term "overexpression" used herein means that the expression of a specific gene into mRNA or the expression level of the gene into protein is significantly up-regulated through intracellular gene transcription or gene translation.

The term "not expressed" used herein means that the expression of a specific gene into mRNA or the expression level of the gene into protein is significantly "downregulated" by intracellular gene transcription or gene translation, so that there is almost no expression level.

In the present disclosure, the expression may be confirmed by measuring the expression level of the gene or mRNA by polymerase chain reaction (PCR), real-time RT-PCR, reverse transcription PCR, competitive RT-PCR, nuclease protection analysis (RNase, S1 nuclease assay), in situ hybridization, nucleic acid microarray, Northern blot, or DNA chip method using a primer pair, a probe, or a primer pair and a probe that specifically recognizes a nucleic acid sequence, a nucleic acid sequence complementary to the nucleic acid sequence, and fragments of the nucleic acid sequence and the complementary sequence. In addition, the expression may be confirmed by measuring the expression level of protein by Western blot, enzyme linked immunosorbent assay (ELISA), radioimmunoassay (RIA), radioimmunodiffusion, immunoelectrophoresis, immunohistochemical staining, immunoprecipitation assay, complement fixation assay, FACS, mass spectrometry, or protein microarray using antibodies, antibody fragments, aptamers, avidity multimers, or peptidomimetics that specifically recognize a full-length protein or fragments thereof.

The term "blood vessel regeneration" used herein means repairing damage to damaged blood vessels, and means promoting angiogenesis, i.e., regeneration of damaged blood vessels, and preventing already damaged blood vessels from being further damaged by continuous external force, etc., using the composition of the present disclosure. The "angiogenesis" or "vasculogenesis" refers to a natural healing process of forming new blood vessels to supply blood to organs or damaged tissues, and the angiogenesis plays an important role in the progression and treatment of many diseases. The vMSCs of the present disclosure have angiogenic ability even in an angiogenic growth factor-deficient medium, and the cells themselves secrete an angiogenic growth factor HGF at a high concentration and overexpress a receptor c-Met thereof, thereby promoting vascular sprouting using HGF as an autocrine factor. In addition, HGF secreted by vMSCs has a paracrine effect that increases the angiogenic ability of peripheral endothelial cells. In addition, bFGF secreted by BM-MSCs promotes vascular sprouting to have a paracrine effect. Therefore, the vMSCs of the present disclosure have blood vessel regeneration, angiogenesis or vasculogenesis effects through an autocrine effect and a paracrine effect.

The term "angiogenesis" used herein may be achieved by the autocrine effect and the paracrine effect of vMSCs. The vMSCs have angiogenic ability even in an angiogenic growth factor-deficient medium, and the cells themselves secrete an angiogenic growth factor HGF at a high concentration and overexpress a receptor c-Met thereof, thereby promoting vascular sprouting using HGF as an autocrine factor. In addition, HGF secreted by the vMSCs promotes vascular sprouting of mature vascular endothelial cells (HUVECs) to have a paracrine effect. In addition, bFGF secreted by BM-MSCs promotes vascular sprouting to have a paracrine effect.

In one aspect, the present disclosure provides a cell therapeutic agent composition for blood vessel regeneration or angiogenesis, including the stem cells of the present disclosure as an active ingredient.

In one embodiment, the composition may further include bone marrow-derived mesenchymal stem cells or adipose-derived mesenchymal stem cells.

In one embodiment, the bone marrow-derived mesenchymal stem cells or adipose-derived mesenchymal stem cells may not express the CD141 cell surface antigen.

In one embodiment, the stem cells (vMSCs) of the present disclosure and the bone marrow-derived mesenchymal stem cells or adipose-derived mesenchymal stem cells may be included in a ratio of 1:10 to 10:1 based on the number of cells, preferably in a ratio of 1:1 to 10:1, more preferably in a ratio of 1:1 to 3:1, and most preferably in a ratio of 2:1.

In one embodiment, the composition may further include HGF or bFGF.

In one embodiment, the blood vessel regeneration may include arterial regeneration, arteriolar regeneration, venous regeneration, capillary vascular regeneration, blood-brain barrier (BBB) regeneration, or retinal vascular regeneration.

In one embodiment, the stem cells may be 3D cultured stem cells.

In one embodiment, the cell therapeutic agent composition may be freeze-stored and used for blood vessel regeneration.

The cell therapeutic agent may be administered to the human body through any common route as long as it may reach a target tissue, and may be administered parenterally or orally, and when administered orally, may be administered by including the cells in a grafting material, etc.

In one aspect, the present disclosure relates to a cell therapeutic agent composition for treating brain neurological disease, including the stem cells of the present disclosure as an active ingredient.

In one embodiment, the brain neurological disease may be a degenerative brain disease, a mental disorder, or a developmental disorder.

In one embodiment, the degenerative brain disease may be cognitive impairment, Alzheimer's disease, dementia with Lewy bodies, frontotemporal dementia, Parkinson's disease, Creutzfeldt-Jakob disease (CJD), Huntington's disease, multiple sclerosis, or Guillain-Barre Syndrome (GBS).

In one embodiment, the mental disorder may be bipolar disorder, autism, depression, hyperactivity, attention deficit, autism, post-traumatic stress disorder (PTSD), anxiety disorder, sleep disorder, panic disorder, intellectual disability, memory impairment, drug addiction, schizophrenia, obsessive-compulsive disorder, delusions of grandeur, personality disorder, alcoholism, or manic depression.

In one embodiment, the developmental disorder may be epilepsy, cerebral palsy, developmental language disorder, disturbance of sense, learning disorder, attention deficit hyperactivity disorder (ADHD), autism spectrum disorder (ASD), intellectual disability, cognitive impairment, or impulse control disorders.

The term "stem cell therapeutic agent" used herein refers to a therapeutic agent used for tissue regeneration treatment by proliferating and selecting living autologous, allogenic, or xenogenic stem cells in vitro and introducing the stem cells into the body to restore the tissue and function of cells.

In one aspect, the present disclosure relates to a graft material for blood vessel regeneration, including the stem cells of the present disclosure or the cell therapeutic agent composition of the present disclosure as an active ingredient.

In one embodiment, the graft material may be surgical implants, medical supplies or medical devices, such as film, membrane, sheet, rod, screw, anchor, pin, implant, stent, surgical suture, scaffold for tissue regeneration, bio-nano fiber, hydrogel, bio-sponge, bone plate and bone graft.

In one embodiment, if the graft material is a hydrogel, the graft material may be administered orally and used for promoting blood vessel regeneration of internal organs.

In one embodiment, the graft material may further include a biodegradable polymer.

In one embodiment, the graft material may be a composite scaffold for tissue engineering, and the composite scaffold for tissue engineering may include the stem cells of the present disclosure or the cell therapeutic agent composition of the present disclosure in a scaffold formed by molding a biodegradable polymer.

In one embodiment, the graft material may be a graft material in which the stem cell or cell therapeutic agent of the present disclosure is inoculated into a composite scaffold for tissue engineering.

In one embodiment, when the graft material is a hydrogel, a polymer forming the hydrogel may be polyethylene glycol (PEG), polyethylene oxide (PEO), polyhydroxyethyl methacrylate (PHEMA), polyacrylic acid (PAA), polyvinyl alcohol (PVA), poly(N-isopropylacrylamide) (PNIPAM), polyvinylpyrrolidone (PVP), polylactic acid (PLA), polyglycolic acid (PGA), polycaprolactone (PCL), gelatin, hyaluronic acid, alginate, carrageenan, chitosan, hydroxyalkyl cellulose, alkyl cellulose, silicone, rubber, agar, carboxyvinyl copolymer, polydioxolane, polyacryl acetate, polyvinyl chloride, or maleic anhydride/vinyl ether.

The term "biodegradable polymer" used herein refers to a polymer that is slowly and spontaneously decomposed in a living body after a certain period of time, and has at least one property of biocompatibility, hemocompatibility, anti-calcification properties, cellular nutrients, and intercellular matrix forming ability. The type of such biodegradable polymer is not particularly limited in the present disclosure, but representative examples thereof may include fibrin, collagen, gelatin, chitosan, alginate, hyaluronic acid, dextran, polylactic acid, poly(glycolic acid) (PGA), poly(lactic-co-glycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymer, copolymers thereof, mixtures thereof, etc. The composite scaffold may be prepared by molding a biodegradable polymer by a conventionally known method, for example, a solvent-casting and particle-leaching technique, a gas forming technique, a fiber extrusion and fabric forming process, a thermally induced phase separation technique, an emulsion freeze drying method, a high pressure gas expansion method, etc.

In one aspect, the present disclosure relates to a pharmaceutical composition for preventing or treating vascular disease or vascular dysfunction, including the stem cells of the present disclosure or the cell therapeutic agent composition of the present disclosure as an active ingredient.

In one embodiment, the vascular disease may be traumatic vascular injury, peripheral vascular disease, cardiovascular disease, cerebrovascular disease, or ischemic disease, and the ischemic disease may be ischemic myocardial infarction, ischemic heart disease, ischemic vascular disease, ischemic enteritis, ischemic eye disease, ischemic glaucoma, ischemic renal failure, ischemic retinopathy, ischemic stroke, or ischemic lower extremity disease.

In one embodiment, the vascular disease or vascular dysfunction may be critical limb ischemia, diabetic angiogenic disorders, vascular dementia, diabetic organ damage, arteriosclerosis, angina, peripheral cardiovascular disease, hypertension, cerebral infarction, brain injury sequelae, heart failure, peripheral circulatory disorders, myocardial infarction, arterial occlusive disease, stroke, spinal cord injury, spinal nerve sequelae, degenerative disease, cerebral infarction sequelae, peripheral neuropathy, diabetic ulcer, presbyopia, degenerative hearing loss, brain surgery sequelae, ischemic stroke, or subarachnoid hemorrhage.

In one embodiment, the composition may induce angiogenesis or vasculogenesis and promote blood vessel regeneration or tissue regeneration, or improve vascular functiona and increase tissue perfusion and new blood vessel formation.

In one aspect, the present disclosure relates to a pharmaceutical composition for preventing or treating brain neurological disease, including the stem cells of the present disclosure or the cell therapeutic agent composition of the present disclosure as an active ingredient.

In one embodiment, the brain neurological disease may be a degenerative brain disease, a mental disorder, or a developmental disorder.

In one embodiment, the degenerative brain disease may be cognitive impairment, Alzheimer's disease, dementia with Lewy bodies, frontotemporal dementia, Parkinson's disease, Creutzfeldt-Jakob disease (CJD), Huntington's disease, multiple sclerosis, or Guillain-Barre Syndrome (GBS).

In one embodiment, the mental disorder may be bipolar disorder, autism, depression, hyperactivity, attention deficit, autism, post-traumatic stress disorder (PTSD), anxiety disorder, sleep disorder, panic disorder, intellectual disability, memory impairment, drug addiction, schizophrenia, obsessive-compulsive disorder, delusions of grandeur, personality disorder, alcoholism, or manic depression.

In one embodiment, the developmental disorder may be epilepsy, cerebral palsy, developmental language disorder, disturbance of sense, learning disorder, attention deficit hyperactivity disorder (ADHD), autism spectrum disorder (ASD), intellectual disability, cognitive impairment, or impulse control disorders.

The pharmaceutical composition of the present disclosure is administered in a pharmaceutically effective amount. As used herein, the 'pharmaceutically effective dose' refers to an amount enough to treat the disease at a reasonable benefit/risk ratio applicable to medical treatment and not to cause side effects. The effective dose level may be determined according to factors including the health condition of a subject, the type and severity of vascular disease or vascular dysfunction, the activity of a drug, the sensitivity to a drug, a method of administration, a time of administration, a route of administration, an excretion rate, duration of treatment, and combined or simultaneously used drugs, and other factors well-known in the medical field. The composition of the present disclosure may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include a carrier, a diluent, an excipient, or a combination of two or more thereof, which are commonly used in biological agents. As used herein, the term "pharmaceutically acceptable" means that the composition exhibits non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present disclosure may further contain one or more active ingredients exhibiting the same or similar functions.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present disclosure is preferably included in an amount of 0.1 part by weight to 90 parts by weight based on the composition, but is not limited thereto.

The composition of the present disclosure may be administered parenterally (e.g., applied intravenously, subcutaneously, intraperitoneally or topically) according to a desired method, and the range of the dose may vary depending on the weight, age, sex, and health condition of a patient, a diet, an administration time, an administration method, an excretion rate, the severity of a disease, etc. A daily dose of the composition according to the present disclosure is 0.0001 to 10 mg/ml, preferably 0.0001 to 5 mg/ml, and more preferably administered once to several times a day.

In one aspect, the present disclosure relates to a method for preparing stem cells expressing a CD141 cell surface antigen, including culturing human bone marrow mononuclear cells (cryopreserved, BM-MNCs) or adipose-derived stroma-vascular fractions (SVFs) in a medium containing a human platelet lysate without containing serum.

In one aspect, the present disclosure relates to a use of stem cells expressing a CD141 cell surface antigen, for use in blood vessel regeneration or angiogenesis.

In one aspect, the present disclosure relates to a method for blood vessel regeneration or angiogenesis, including administering stem cells expressing a CD141 cell surface antigen to a subject.

In one aspect, the present disclosure relates to a method for treating vascular disease or vascular dysfunction, including administering stem cells expressing a CD141 cell surface antigen to a subject suffering from vascular disease or vascular dysfunction.

In one aspect, the present disclosure relates to a method for treating brain neurological disease, including administering stem cells expressing a CD141 cell surface antigen to a subject suffering from brain neurological disease.

### [Modes]

Hereinafter, the present disclosure will be described in more detail with reference to the following Examples. However, the following Examples are only intended to embody the contents of the present disclosure, and the present disclosure is not limited thereto.

### Example 1. Isolation of novel multipotent stem cells derived from bone marrow

### 1-1. Optimal culture of vMSCs

Cryopreserved human bone marrow mononuclear cells (BM-MNCs) (STEMCELL Technologies, Vancouver, Canada, LONZA, Basel, Switzerland) were thawed at 37°C, or a bone marrow aspirate obtained with patient consent was subjected to density gradient centrifugation with Ficoll-paque (Cytiva, MA, USA) to obtain BM-MNCs. The BM-MNCs obtained above were dispensed at a density of 1.0 to 2.0 × 10⁵ cells/cm² in a TC-treated (tissue culture treated) flask using an FBS (Fetal bovine serum)-deficient EGM-2 bulletkit medium (Lonza; Basel, Switzerland) (EGM-2-FBS+2%hPL) supplemented with 2% human platelet lysate (PL) (PL bioscience, GmbH, Germany) and 2 unit/mL heparin (Sigma-Aldrich, St. Louis), and then primary-cultured for 7 to 12 days. During the primary culture period, medium replacement was performed once every 1 to 4 days. Colonies of the novel multipotent stem cells of the present disclosure, Vasculogenic Multipotent Stem Cells (vMSCs), begin to be observed from 3 to 5 days of culture, and the vMSCs cells forming the colonies observed during the primary culture had a spindle shape and were very diverse in length and size, and it was observed that the colonies expanded significantly between 7 and 10 days. The first subculture period of vMSCs was performed when the majority of colonies showed a density of 80 to 90% or more, and the vMSCs were isolated into single cells using trypsin or a trypsin substitute in the same manner as the subculture method of general adherent cells, and then the isolated cells were dispensed into a new flask at 1.0 to 5.0 × 10³ cells/cm² and subcultured when the density was 70 to 90%, and then subcultured 5 to 7 times. As such, the vMSCs of the present disclosure were cultured by excluding FBS from EGM-2 and adding a human platelet lysate (PL), unlike endothelial progenitor cells (EPCs) that were cultured with EGM-2 containing FBS using basic components of an EGM-2 kit as they were.

### 1-2. Confirmation of extracellular matrix adhesion properties of vMSCs

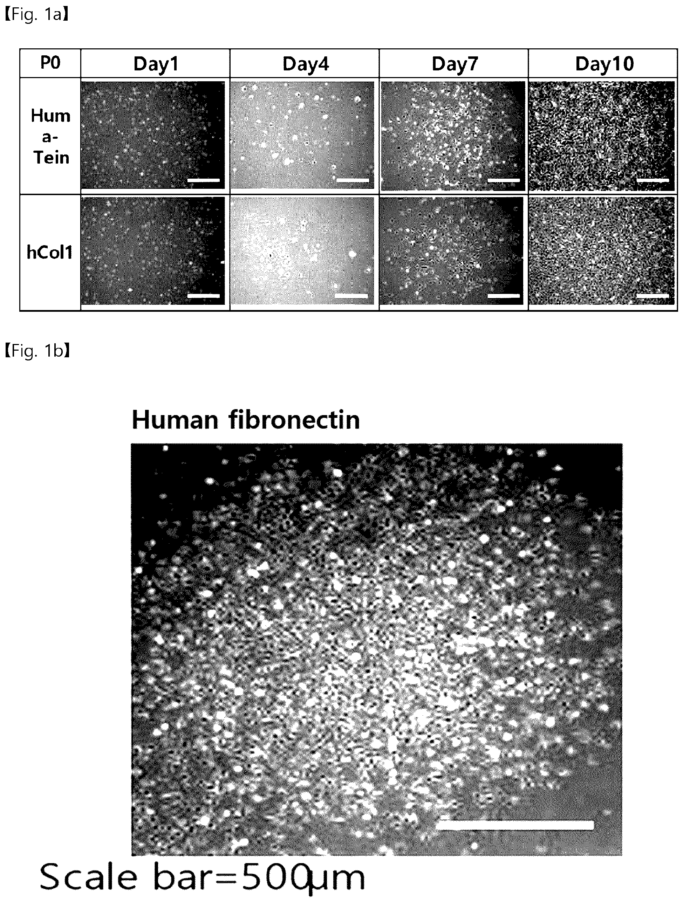

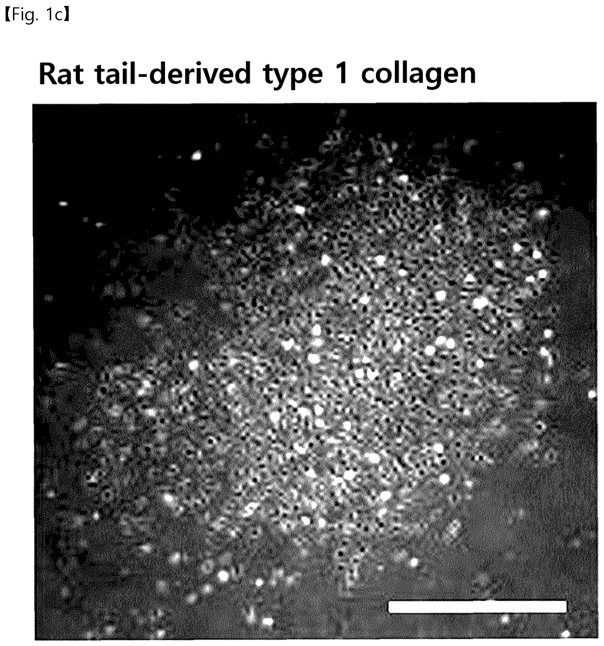

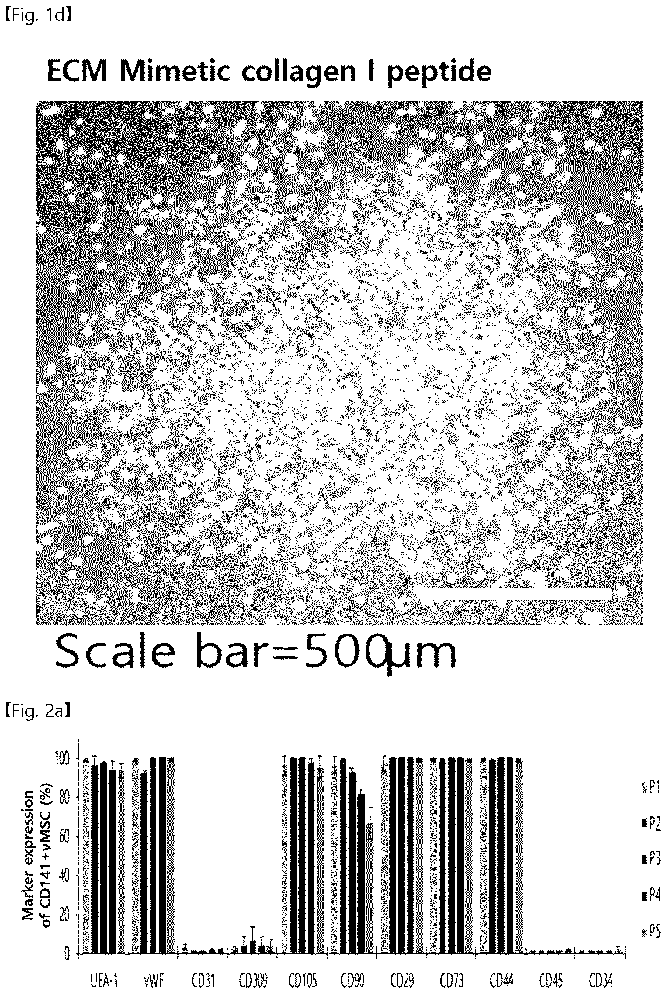

The vMSCs were cultured and identified in a culture flask coated with various extracellular matrices (ECMs), including a human fibroblast-derived ECM complex, HumaTein (100 µg/mL, ROKIT healthcare, Seoul, Republic of Korea), human type I collagen (0.2 µg/cm²) (Corning, NY, USA), Fibronectin (1 µg/cm²) (Sigma-Aldrich), porcine collagen type 1-P (Cellmatrix Type I-P) (Nitta Gelatin, Osaka, Japan), collagen type I from rat tail (50-825 µg/mL) (ThremoFisher, MA, USA), and collagen type I peptide (Corning). As a result, the cells forming the vMSC colonies were identically shown in a spindle shape on all coating materials, and could be cultured in various extracellular matrices. When the cells were cultured in the ECM-coated flask as described above, more colonies were obtained than in a general TC-treated flask, but there was no significant difference (FIG. 1). That is, although the primary culture yield may vary depending on the presence and type of ECM coating, there was little difference in the proliferation rate after P1 depending on the type and presence of coating.

The novel vMSCs of the present disclosure cannot maintain the characteristics of CD141⁺vMSCs when cultured using FBS of an existing EGM-2 bullet kit instead of human PL, and the addition of human PL further increased the expression of CD141. Therefore, the culture solution thereof essentially added human PL to an EGM-2 bullet kit (Lonza) and excluded FBS, and there was no problem with growth even if IGF and VEGF were excluded, and thus IGF and VEGF can be excluded.

### Example 2. Marker characteristic analysis of vMSCs

### 2-1. Confirmation of expression of EPC and endothelial cell markers

The expression of markers of conventional EPCs and endothelial cells in vMSCs of the present disclosure was confirmed by flow cytometry, and the expression of eNOS and VE-cadherin expressed by mature endothelial cells was confirmed by immunofluorescence staining analysis, and compared with bone marrow mononuclear cells (BM-MSCs) and human umbilical cord endothelial cells (HUVECs), respectively. Specifically, for flow cytometry, vMSCs and BM-MSCs were resuspended in a PEB buffer containing 2 mM EDTA and 0.5% bovine serum albumin (BSA) (Sigma-Aldrich), respectively, and then blocked with a human FcR blocking reagent (Miltynyi biotec) at 4°C for 10 minutes. Thereafter, the vMSCs and BM-MSCs were stained with Alexa flour 488-conjugated vWF antibody (Abcam, 1:500), fluorescein-labeled UEA-1 (Vectorlabs, 3:100), and allophycocyanin (APC)-conjugated CD29, CD31, CD34, CD44, CD45, CD73, CD90, CD105, and CD309 antibodies (Miltenyl Biotec, 1:50), respectively. At this time, Isotype IgG conjugated with APC (Miltenyl Biotec. 1:50) and Alexa flour 488 (Abcam, 1:500) was used as a control group. After antibody staining, the cells were washed with a PEB buffer and centrifuged at 1,000 ×g and 4°C for 5 minutes. Finally, the cells were resuspended in a PEB buffer and analyzed using a NovoCyte 3000 flow cytometer (Agilent Technologies, Santa Clara, CA, USA) and NovoExpress software. In addition, for immunofluorescence staining analysis, vMSCs and HUVECs were cultured on coverslips, respectively, and fixed with 3.7% formaldehyde (Sigma-Aldrich), and then permeated into the cell membrane with 0.2% Triton X-100. For blocking, the cells reacted with 20% normal goat serum (NGS) for 1 hour at room temperature, and then reacted with primary antibodies, e-NOS (Cell Signaling Technology, MA, USA, 1:400) and VE-cadherin (Cell Signaling Technology, 1:400), diluted in 20% NGS, at 4°C overnight, respectively. The next day, the cells reacted with an Alexa fluor 488-conjugated anti-rabbit secondary antibody (Invitrogen, MA, USA, 1:1000) diluted in 20% NGS for 1 hour at room temperature. After mounting with a mounting solution containing DAPI (Vector Laboratories, CA, USA) for sample observation, images were obtained using a fluorescence microscope (Leica).

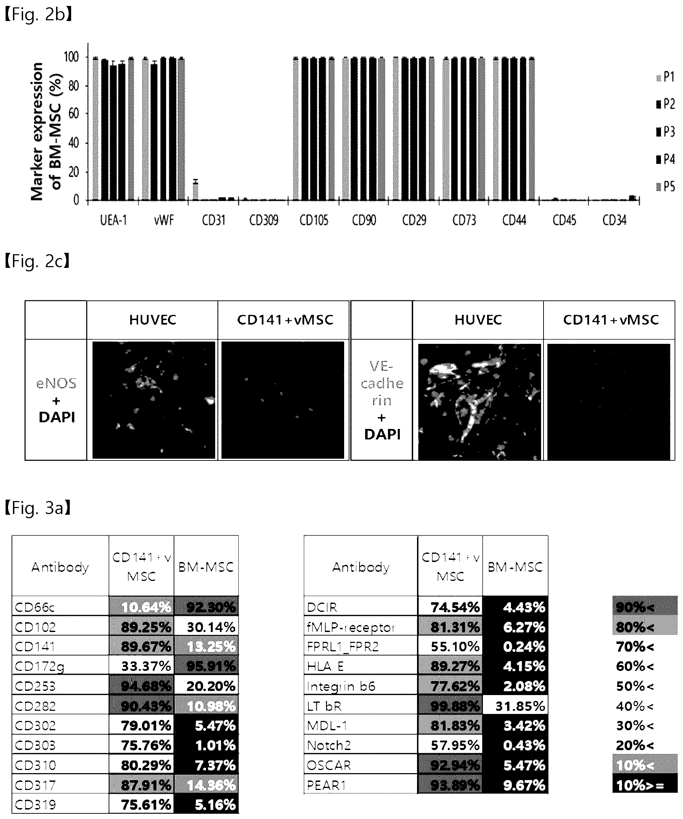

As the flow cytometry result, it was shown that the vMSCs of the present disclosure expressed all of CD105, CD90, CD29, CD73, and CD44, which were known as MSC markers, and did not express CD31, CD309, and CD34, which were commonly expressed by previously reported EPCs (FIG. 2A). In addition, as the result of immunofluorescence staining analysis, it was shown that the vMSCs of the present disclosure did not express eNOS and VE-cadherin, unlike HUVECs, as a positive control group (FIG. 2C). Through this, it can be seen that vMSCs share more markers with MSCs than EPCs.

### 2-2. Discovery of novel vMSC-specific markers

Marker screening was conducted to discover vMSC-specific markers of the present disclosure that were distinguished from BM-MSCs. To this end, MACS^{®}Marker Screen, human, version 02 (Miltenyi Biotec), capable of analyzing 378 surface antigen markers by flow cytometry, was used, and the experiment was performed according to the manufacturer's instructions. Specifically, vMSCs (5.7 × 10⁷) and BM-MSCs (5.7 × 10⁷) at Passage 3 derived from the same donor were resuspended in a PEB buffer and blocked with a human FcR blocking reagent (Miltenyi Biotec) at 4°C for 10 minutes. Thereafter, 1.2 × 10⁵ /well of cells were dispensed into each of four 96-well plates containing six isotype controls and 378 antibodies, and then reacted at 4°C for 20 to 30 minutes. The cells were washed with a PEB buffer, centrifuged at 300 × g for 5 minutes, resuspended in PEB buffer at a concentration of 200 µl/well, and analyzed using a NovoCyte 3000 flow cytometer (Agilent Technologies) equipped with a NovoSampler (Agilent Technologies) and NovoExpress software.

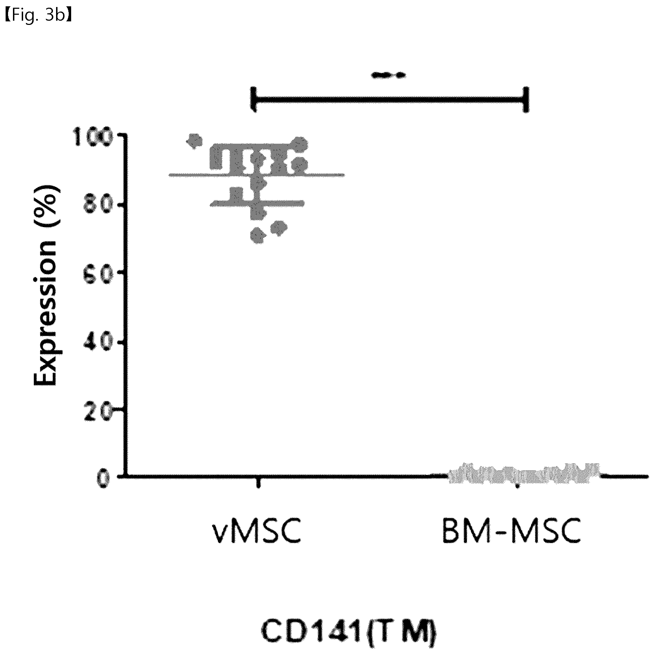

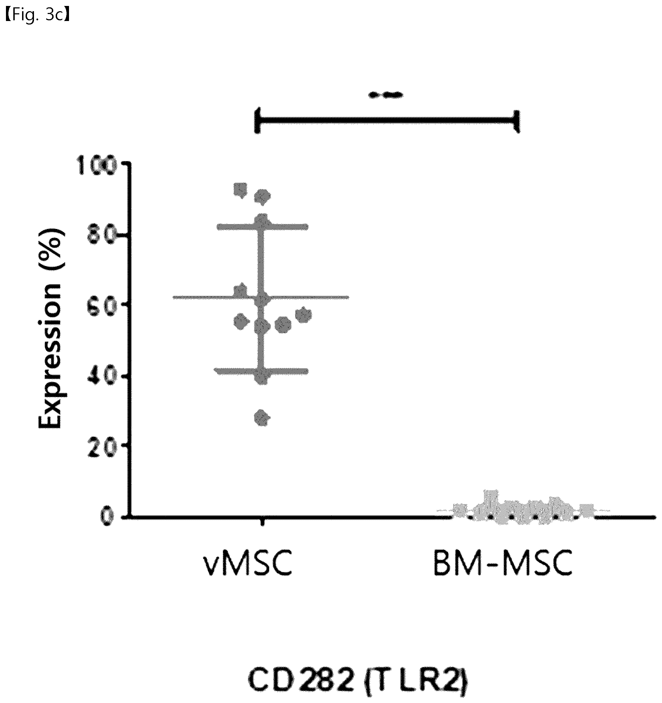

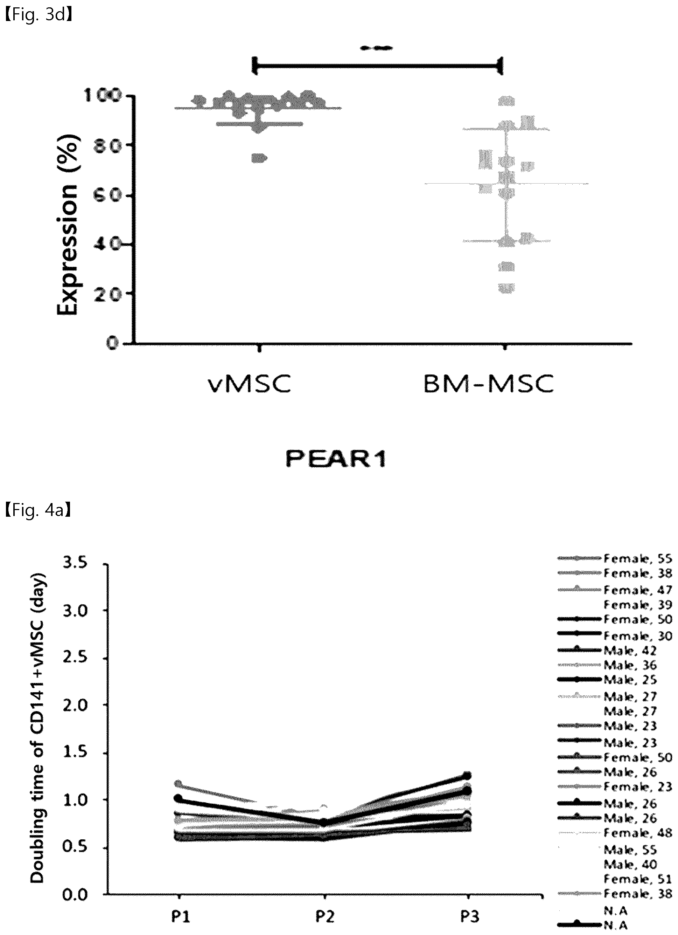

As a result of marker screening, about 21 markers that showed a difference between vMSCs and BM-MSCs were selected (FIG. 3A), and for validation, flow cytometry was performed for CD141, CD282, and PEAR1 (Platelet Endothelial Aggregation Receptor 1) in vMSCs and BM-MSCs derived from at least 10 donors using APC-conjugated CD141, CD282, and PEAR1 (Miltenyi biotec. 1:50). As a result, CD141 was consistently shown to be expressed at 70 to 99% in vMSCs, but not expressed in BM-MSCs (FIG. 3B). In addition, CD282 was not expressed in BM-MSCs, but was expressed in vMSCs at levels from 30% to 90%, showing a large difference between donors (FIG. 3C). In addition, PEAR1 was expressed at 70 to 99% in vMSCs, but expressed from 20% to 90% in BM-MSCs, showing a large difference between the donors (FIG. 3D). Through this, it was shown that CD141 was a clear marker that distinguished between vMSCs and BM-MSCs of the present disclosure.

### Example 3. Analysis of cell expansion ability of vMSCs

To compare and evaluate the cell expansion ability of CD141⁺vMSCs with that of BM-MSCs, the CD141+vMSCs were cultured up to Passage 3 from at least 20 donors using the method described in Example 1, and simultaneously with the primary culture of CD141⁺vMSCs, BM-MNCs were dispensed at 4.0 to 7.0 × 10⁴ cells/cm² in StemMACS^{™} expansion media, and XF (Miltenyi Biotec, Bergisch Gladbach, Germany) (StemMACS) medium, and then primary-cultured for the same period as CD141⁺vMSCs, and subcultured at the same time as CD141⁺vMSCs. After culture in this manner, the population doubling time (PDT) per passage of the CD141⁺vMSC and BM-MSC and the number of primary cultured MNCs were fixed to 1 × 10⁷, and the cumulative cell number up to Passage 3 was measured.

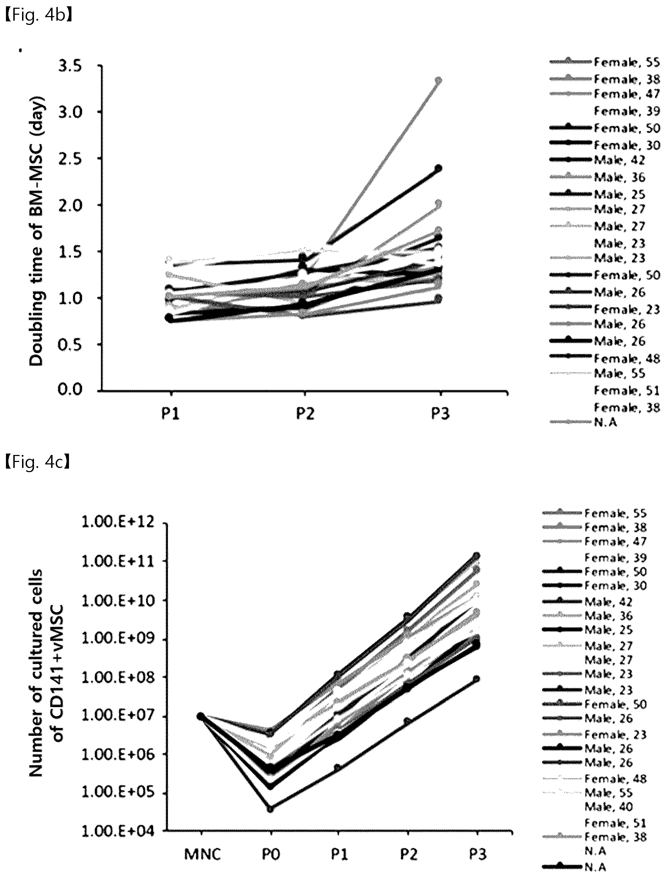

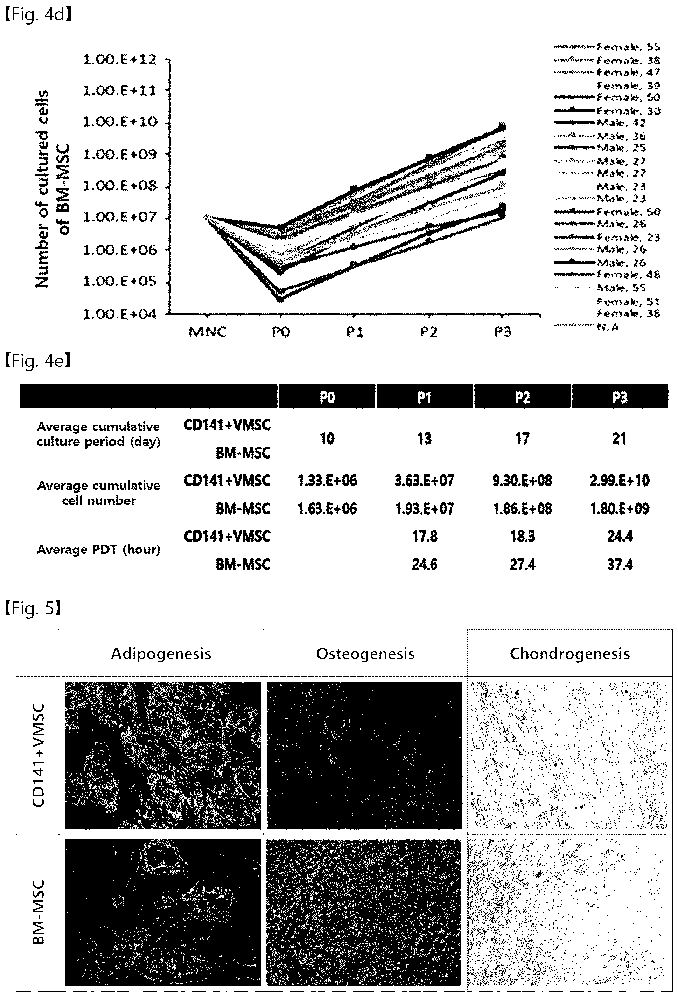

As a result, the average PDT from P1 to P3 was 0.84 days (20 hours) for CD141⁺vMSCs and 24 days (30 hours) for BM-MSCs, indicating that CD141⁺vMSCs divided approximately 1.5 times faster (FIGS. 4A and 4B). In addition, as a result of confirming the cumulative cell number, 3.0 x10¹⁰ cells could be obtained within Passage 3 (19 to 22 days) for CD141⁺vMSCs, and approximately 1.8 x10⁹ cells could be obtained for BM-MSCs (FIGS. 4C and 4D). That is, CD141⁺vMSCs were able to obtain 16 to 17 times higher numbers of cells than BM-MSCs, indicating that CD141⁺vMSCs were cells with very excellent cell expansion ability (FIG. 4E).

### Example 4. Analysis of multipotency of vMSCs

### 4-1. Adipogenesis differentiation characteristics

To confirm the multipotency of CD141⁺vMSC, Passage 3 CD141⁺vMSC and BM-MSC derived from the same donor were cultured in an adipogenesis differentiation inducing medium. Specifically, CD141⁺vMSCs were dispensed at 1.0 × 10³/cm² in an EGM-2-FBS+2%hPL medium and BM-MSCs were dispensed at 1.0 × 10³/cm² in a StemMACS medium in 6-well plates, and when the cells reached almost 100% density, the media were replaced with StemPro^{™}Adipogenesis Differentiation Kit (Gibco) media, which were adipogenesis differentiation inducing media, and the cells were cultured for 14 days by replacing the medium once every 3 to 4 days. After fixing each cell with 3.7% formaldehyde (Sigma-Aldrich), the cells were reacted with 60% isopropanol (Daejung, Sihueng si, Republic of Korea) at room temperature for 5 minutes, and then stained with a 0.3% Oil red O (Sigma-Aldrich) solution containing 60% isopropanol for 20 minutes at room temperature. Thereafter, the cells were washed with distilled water and images were obtained using a microscope (Nikon, Tokyo, Japan). As a result, CD141⁺vMSCs were shown to differentiate into adipose like MSCs (FIG. 5).

### 4-2. Osteogenesis differentiation characteristics

To confirm the multipotency of CD141⁺vMSC, Passage 3 CD141⁺vMSC and BM-MSC derived from the same donor were cultured in an osteogenesis differentiation inducing medium. Specifically, CD141⁺vMSCs were dispensed at 1.0 × 10³/cm² in an EGM-2-FBS+2%hPL medium and BM-MSCs were dispensed at 1.0 × 10³/cm² in a StemMACS medium in 6-well plates, and when the cells reached 80 to 90% density, the media were replaced with StemPro^{™}Osteogenesis Differentiation Kit (Gibco) media, which were osteogenesis differentiation inducing media, and the cells were cultured for 21 days by replacing the medium once every 3 to 4 days. After fixing each cell with 3.7% formaldehyde (Sigma-Aldrich), the fixed cells were stained with a 2% Alizarin red S (Sigma-Aldrich) solution for 20 minutes at room temperature. Thereafter, the cells were washed with distilled water and images were obtained using a microscope (Nikon, Tokyo, Japan). As a result, CD141⁺vMSCs were shown to undergo osteogenesis differentiation like MSCs (FIG. 5).

### 4-3. Chondrogenesis differentiation characteristics

To confirm the multipotency of CD141⁺vMSC, Passage 3 CD141⁺vMSC and BM-MSC derived from the same donor were cultured in a chondrogenesis differentiation inducing medium. Specifically, CD141⁺vMSCs were dispensed at 1.0 × 10³/cm² in an EGM-2-FBS+2%hPL medium and BM-MSCs were dispensed at 1.0 × 10³/cm² in a StemMACS medium in 6-well plates, and when the cells reached almost 100% density, the media were replaced with StemPro^{™}Chobdrogenesis Differentiation Kit (Gibco) media, which were chondrogenesis differentiation inducing media, and the cells were cultured for 21 days by replacing the medium once every 3 to 4 days. After fixing each cell with 3.7% formaldehyde (Sigma-Aldrich), the fixed cells were reacted with 3% acetic acid (Sigma-Aldrich) for 3 minutes at room temperature and then stained with 1% alcian blue for 30 minutes at room temperature. Thereafter, the cells were washed with distilled water and images were obtained using a microscope (Nikon, Tokyo, Japan). As a result, CD141⁺vMSCs were shown to differentiate into cartilage like MSCs (FIG. 5).

Through this, it was confirmed that the novel CD141⁺vMSCs of the present disclosure possessed multipotency that has not been reported in existing EPCs and differentiated into fat, bone, and cartilage like MSCs.

### Example 5. Analysis of blood vessel regeneration ability of vMSCs

To compare the blood vessel regeneration ability of CD141⁺vMSCs prepared in Example above with BM-MSCs and mature vascular endothelial cells, HUVECs, a Matrigel tube formation assay was performed. Specifically, Matrigel (Corning Inc., NY, USA) was treated on µ-slides (ibidi, Grafelfing, Germary) at 10 µl per well, and CD141⁺vMSCs, BM-MSCs, and HUVECs were suspended in an α-MEM (GIBCO, NY, USA) medium lacking growth factors and containing 0.2% hPL or an EGM2 medium containing proangiogenic growth factors such as VEGF and FGF, respectively, and dispensed on Matrigel at 6,000 cells/well, and cultured overnight (at least 16 hours). Thereafter, images were obtained with a microscope (Leica, Wetzlar, Germany).

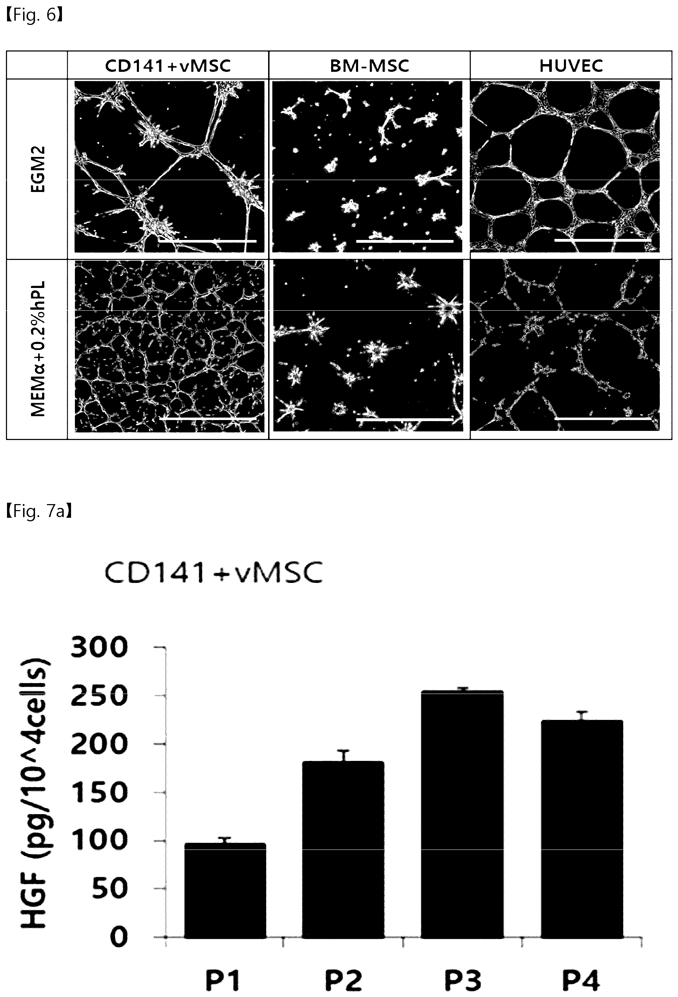

As a result, the BM-MSCs did not form vascular network structures in the two media, and thevascular endothelial cells, HUVECs formed vascular network structures very well in the EGM2 medium containing the proangiogenic growth factors, but did not maintain vascular network structures in the MEMα+0.2% hPL medium without proangiogenic growth factors. In contrast, CD141+vMSCs of the present disclosure formed very thick and large vascular network structures in the EGM2 medium, and formed small and many vascular network structures in the MEMα+0.2% hPL medium (FIG. 6). That is, CD141+vMSCs were shown to have angiogenic ability even in the medium lacking the proangiogenic growth factors.

### Example 6. Analysis of angiogenic growth factor secretion ability of vMSCs

### 6-1. Analysis of HGF secretion ability of vMSCs

To confirm the secretion ability of a Hepatocyte growth factor (HGF), a potent angiogenic growth factor as well as wound healing, an enzyme-linked immunosorbent assay (ELISA) was performed. Specifically, as in Example above, CD141⁺vMSCs and BM-MSCs were dispensed at a concentration of 9 × 10⁴ cells/well in a 6-well plate using each culture medium (EGM-2-FBS+2%hPL and StemMACS), and to exclude a difference due to the medium, the next day, the medium was replaced with MEMα+0.2%hPL, a medium lacking growth factors, and cultured for 48 hours. Thereafter, the culture medium was collected and the absorbance was measured at 450 nm using a microplate reader (Molecular Device, SpectraMax ABS) according to the manufacturer's instructions using Quantikine HGF ELISA (R&D systems, Minneapolis, USA), and then the amount of HGF secreted per cell was analyzed by dividing the absorbance by the number of cells. As such, the cells were cultured up to Passage 4, and the culture supernatant was collected just before subculture, and the secretion level of HGF was analyzed.

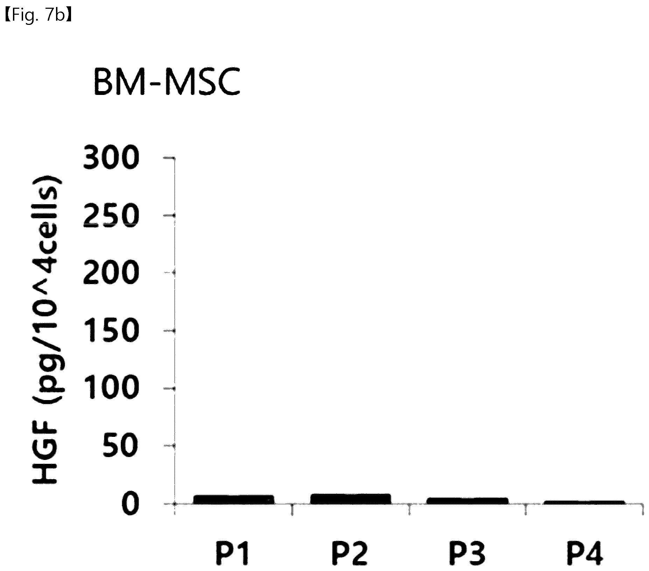

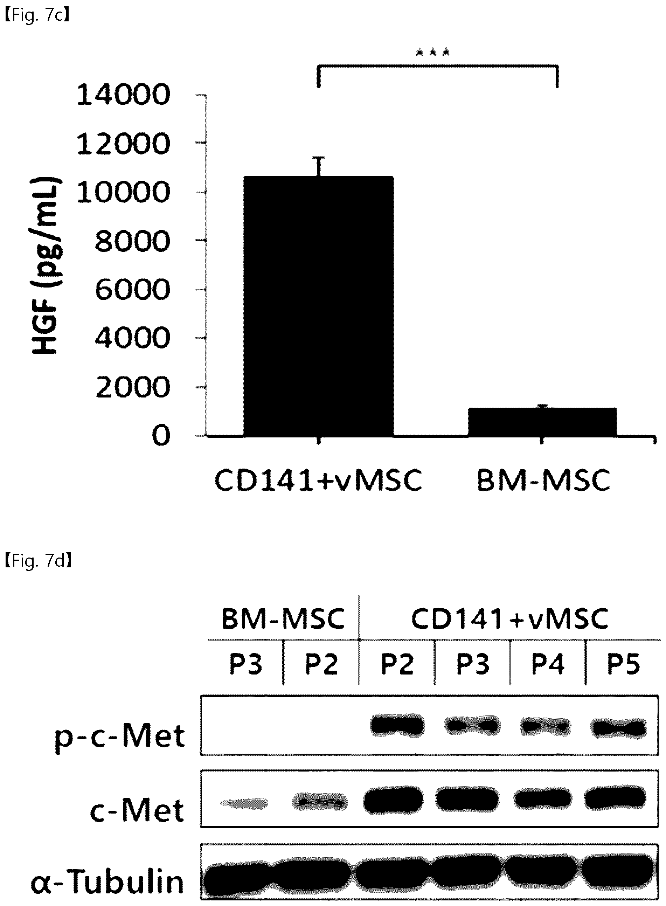

As a result of comparing CD141⁺vMSCs and BM-MSCs of the same passage, it was shown that CD141⁺vMSCs secreted 14 to 130 times more HGF than BM-MSCs (FIGS. 7A and 7B), and under the same conditions, CD141⁺vMSCs secreted about 9 times more HGF than BM-MSCs (FIG. 7C).

### 6-2. Analysis of HGF receptor expression in vMSCs

As Example above, CD141⁺vMSCs and BM-MSCs were cultured and the cells obtained by passage were dissolved using 2 mM PMSF (phenylmethylsulphonyl fluoride, Sigma-Aldrich) and lysis buffer (Cell signaling tech.), centrifuged at 14,000 xg at 4°C for 10 minutes, and only the supernatant was collected to obtain protein lysates. The protein lysates adjusted to the same concentration using a BCA assay (Thermo Fisher Scientific) were separated by SDS-PAGE and then transferred to a nitrocellulose membrane. After blocking the membrane for 1 hour at room temperature with TBS-T containing 5% skim milk, the membrane was reacted overnight at 4°C with antibodies against c-Met (cell signaling tech, 1:1000), phosphor-c-Met (cell signaling, 1:1000), and α-tubulin (Sigma-Aldrichi, 1:4000), respectively. The next day, the membrane was reacted with HRP-conjugated secondary antibody for 1 hour at room temperature, and the membrane after antibody reaction was treated with EZ-western Lumi pico (Dogen, Seoul, Korea) to be luminescent. Thereafter, signals were detected with a Chemilluminator and quantified with Image J software to confirm the expression level of c-Met, an HGF receptor, and its activation (phosphorylation) by passage.

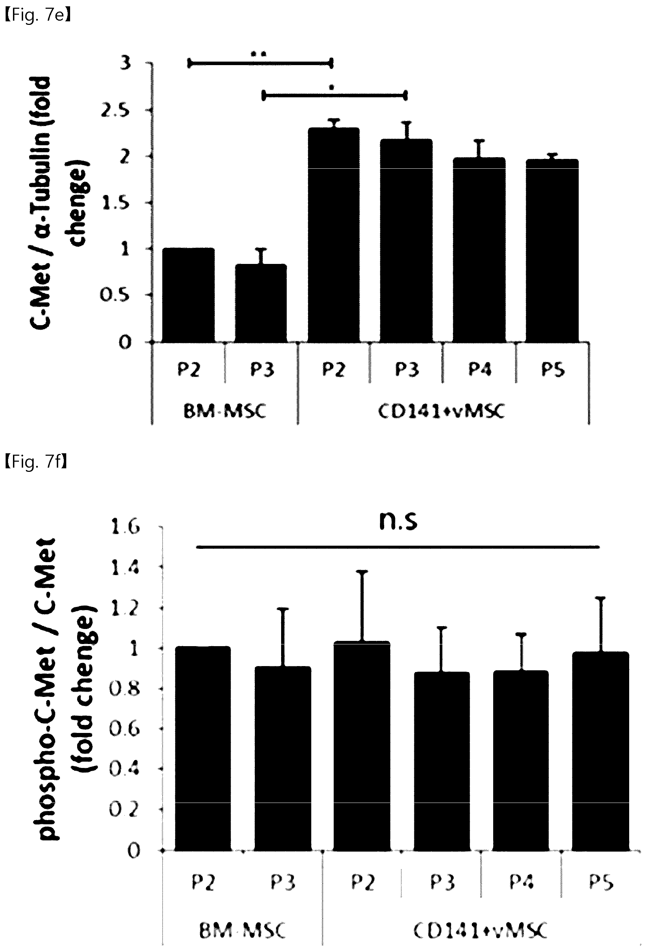

As a result, CD141⁺vMSC cells (CD141⁺vMSC) were shown to express the HGF receptor (c-Met) significantly more than BM-MSC cells (FIGS. 7D and 7E). There was no difference in activity between the two cells (FIGS. 7D and 7F). Therefore, it was confirmed that CD141⁺vMSCs were cells not only secreting a large amount of HGF themselves, but also expressing a large amount of HGF receptor (c-Met) to utilize HGF as an autocrine factor.

### 6-3. Confirmation of angiogenesis mechanism

### 6-3-1. Confirmation of angiogenic sprouting capacity by autocrine factors

To confirm an angiogenic mechanism of CD141⁺vMSC, the angiogenic sprouting capacity by HGF was compared and analyzed with that of BM-MSC. To this end, CD141⁺vMSC spheroids and BM-MSC spheroids were prepared using a hanging drop method, and then treated with HGF or an HGF-neutralizing antibody for HGF inhibition to perform an in vitro spheroid sprouting assay. Specifically, CD141⁺vMSCs and BM-MSCs (2 × 10⁴ cells/mL) were suspended in an M199 medium containing 10% FBS (Thermo Fisher Scientific, Waltham, MA, USA), 1% P/S, and 0.2% methylcellulose (Sigma-Aldrich, St. Louis, Mo, USA), and the suspended cells were inoculated in the form of droplets on the cover of a petri dish in a dose of 30 µL. The inoculated cover of the petri dish was turned over and cultured in a 37°C, 5% CO₂ incubator for 24 hours to form spheroids. After 24 hours of culture, the formed CD141⁺vMSC and BM-MSC spheroids were mixed with 3 mg/mL Collagen type I-A (final concentration 1.44 mg/mL; Nitta Gelatin) and methylcellulose (final concentration 0.48%; Sigma-Aldrich) (Collagen gel mixture) and dispensed by 0.7 mL into 24-well plates, respectively. The collagen gel mixture containing the spheroids was polymerized in a 37°C and 5% CO₂ incubator for 30 minutes. At this time, for the HGF neutralizing antibody treatment group, 500 ng/mL of HGF neutralizing antibody (MAB294, R&D systems; Anti-HGF Ab) was mixed with the collagen gel mixture containing each spheroid and then polymerized (3 hours before HGF treatment). After polymerization, HGF (Peprotech, New Jersey, USA) and/or HGF-neutralizing antibody (control group: 500 ng/mL mouse IgG, IgG) were diluted in an M199 (Sigma-Aldrich) medium and treated with 100 µl each onto the collagen gel mixture, cultured for 48 hours, and sprouting photographs of the spheroids were taken and obtained with a microscope (Nikon). The number of sprouts, the average sprout length, and the total sprout length of the spheroids were measured using Image J software (NIH).

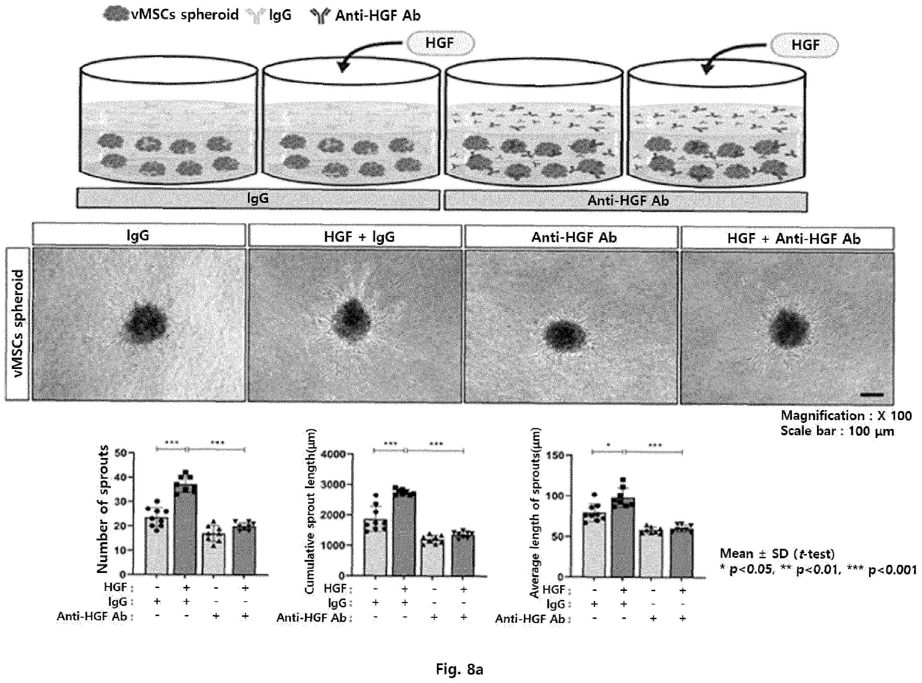

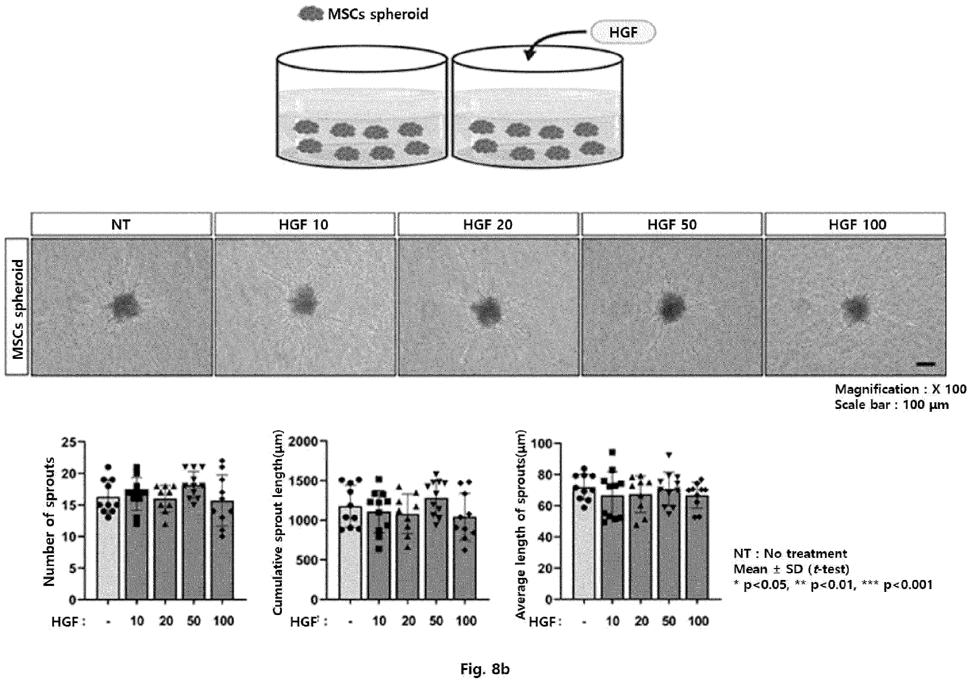

As a result of quantitative analysis of the angiogenic sprouting capacity, it was shown that in the vMSC spheroids of the present disclosure, the vascular sprouting was significantly increased by HGF treatment, and the vascular sprouting was reduced when the HGF and HGF neutralizing antibody were simultaneously treated (FIG. 8A). In contrast, BM-MSC (MSCs) spheroids showed no change in vascular sprouting according to treatment with the HGF or its neutralizing antibody (FIG. 8B).

That is, unlike BM-MSCs that have HGF-independent sprouting ability, it can be seen that CD141⁺vMSCs have HGF-dependent sprouting ability, secrete a lot of HGF by themselves, and express a lot of c-Met to be sprouted using HGF as an autocrine factor.

### 6-3-2. Comparison of angiogenic sprouting capacity by BM-MSC secreted bFGF

To confirm the angiogenic mechanism of CD141⁺vMSC, as in Example 6-3-1 above, BM-MSC and/or a bFGF receptor inhibitor PD173074 (Tocris, Bristol, UK) were diluted in an M199 medium (Sigma-Aldrich) and treated with 100 µl each onto the collagen gel mixture during an in vitro spheroid sprouting assay. After culturing for 48 hours, an effect of bFGF secreted by BM-MSC on the spheroid sprouting of CD141⁺vMSC was confirmed.

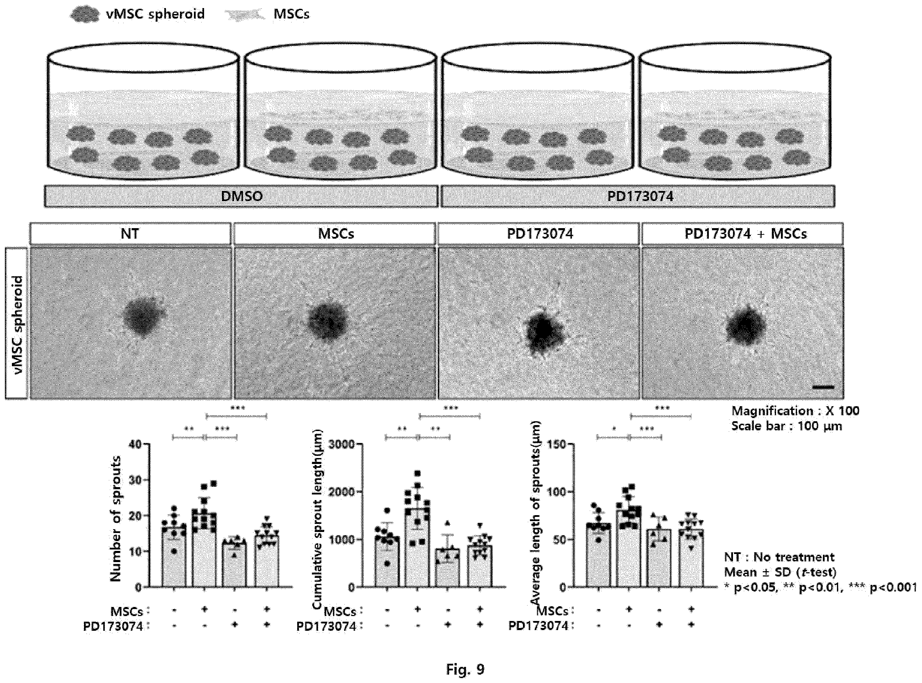

As a result, compared to an untreated group (NT) of CD141⁺vMSC spheroids, when the CD141⁺vMSC spheroids were cultured with BM-MSCs, it was shown that the vascular sprouting ability of CD141⁺vMSCs was increased (FIG. 9). When treated with PD173074, a bFGF receptor inhibitor, it was shown that the vascular sprouting ability of CD141⁺vMSCs increased by BM-MSCs was decreased (FIG. 9).

That is, it was inferred that the bFGF receptor inhibitor PD173074 inhibited a paracrine effect of BM-MSCs on the vascular sprouting of vMSCs, and that a synergistic effect on angiogenesis would occur when the CD141⁺vMSCs of the present disclosure were used in combination with HGF, bFGF or BM-MSCs.

### 6-3-3. Confirmation of angiogenic sprouting capacity by VEGF

To confirm the angiogenic mechanism of CD141⁺vMSC, it was confirmed whether the angiogenic sprouting capacity of CD141⁺vMSC spheroids and BM-MSC spheroids was changed by VEGF, an important angiogenic factor, through the in vitro spheroid sprouting assay as described above, and the mRNA expression of VEGFR2 in BM-MSCs and vMSCs was compared with that of HUVEC cells, a type of EPC.

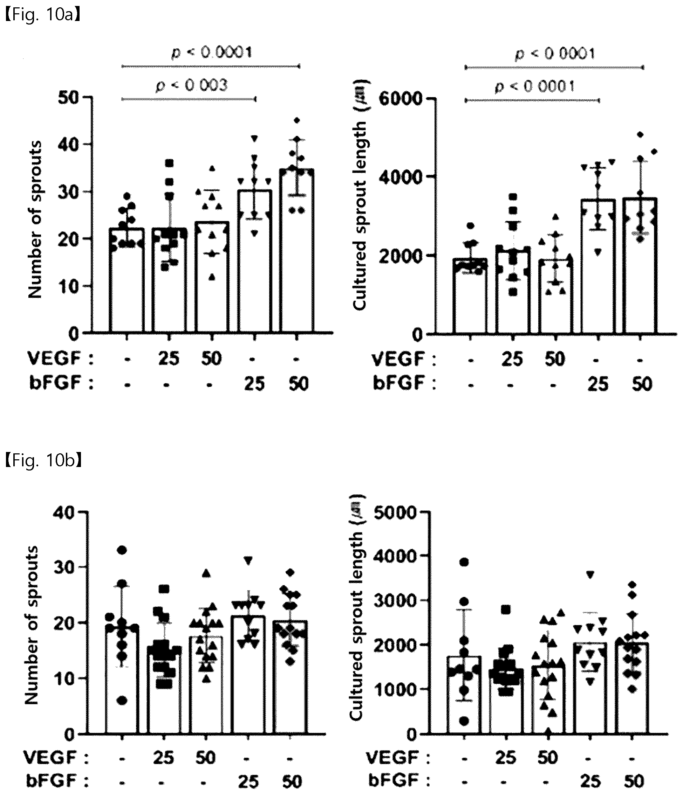

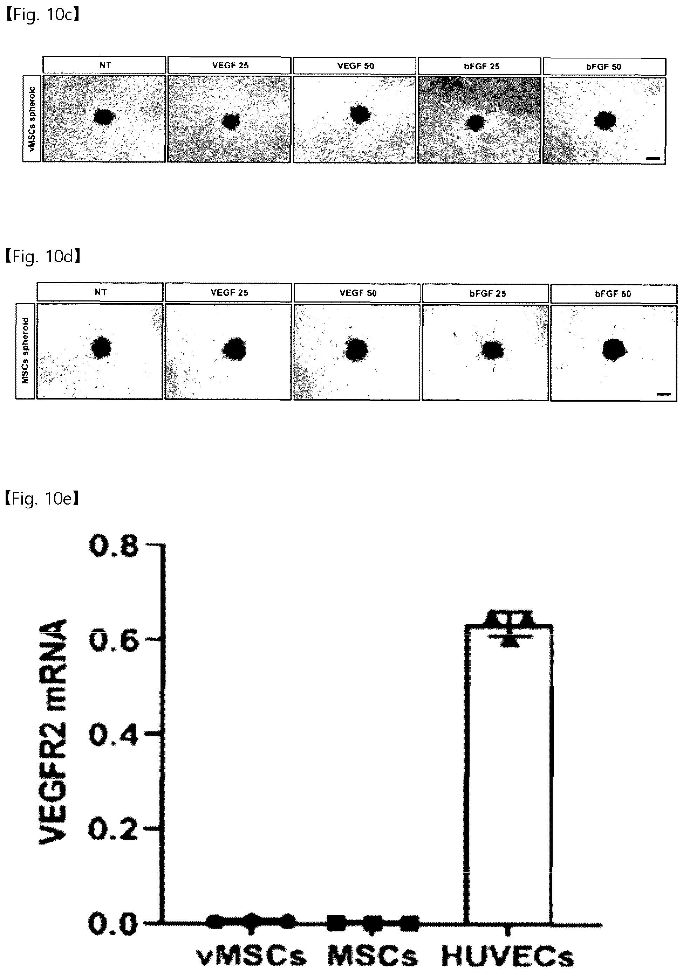

As a result, CD141⁺vMSC spheroids and BM-MSC spheroids did not respond to VEGF (FIGS. 10A to 10D), which was consistent with a result that BM-MSCs and CD141⁺vMSCs did not express VEGF receptors (FIG. 10E). In addition, it was shown that by bFGF treatment, CD141⁺vMSC spheroids had increased angiogenic sprouting capacity, but BM-MSC spheroids did not show increased angiogenic sprouting capacity (FIGS. 10A to 10D).

Through this, it was found that the sprouting of EPC and endothelial cells was promoted by VEGF, but CD141⁺vMSC did not promote vascular sprouting by VEGF and did not express a VEGF receptor 2 (VEGFR2). In addition, it can be seen that the promotion of sprouting by bFGF is a characteristic of CD141⁺vMSC of the present disclosure that is different from BM-MSC.

### 6-3-4. Confirmation of angiogenic paracrine effect of vMSC-secreted HGF

To confirm a paracrine effect of vMSCs on promoting angiogenesis of endothelial cells, HUVEC cells were cultured into spheroids using a hanging drop method as in Example 6-3-1, and contactless co-cultured with vMSCs, and then a spheroid sprouting assay was performed. At this time, to neutralize HGF among secretomes of vMSCs, 500 ng/mL of HGF neutralizing antibody (MAB294, R&D systems; Anti-HGF Ab) was mixed in the M199 medium and polymerized, and 100 µl of the mixture was treated on a collagen gel containing the HUVEC spheroids and cultured for 24 hours, and mouse IgG (IgG) (500 ng/mL) was used as a control group. In sprouting images of the spheroids, the number of sprouts, the cumulative sprout length (sum of all sprout lengths formed from one spheroid), and the average sprout length (average of all sprout lengths formed from all spheroids) were quantitatively analyzed using Image J software (NIH).

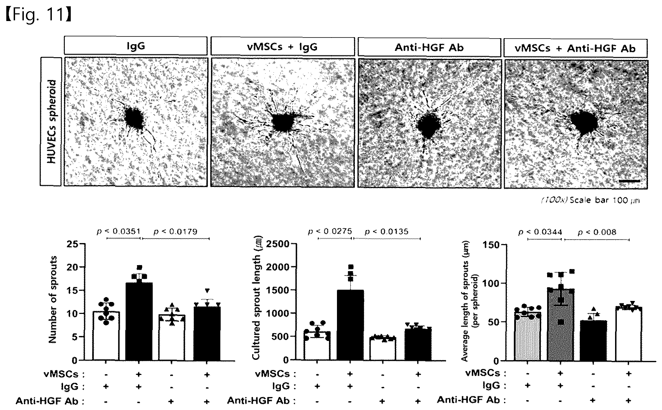

As a result, it was shown that a sprouting-promoting effect on HUVEC spheroids was exhibited during contactless co-culture with CD141⁺vMSCs, and that the HUVEC sprouting effect was inhibited by treatment with the HGF-neutralizing antibody (FIG. 11). Through this, it was confirmed that HGF, among the secretomes of vMSC, was a substance that exhibited a cell sprouting effect on vascular endothelial cells (HUVEC), that is, promoted angiogenesis.

Therefore, it can be seen that bone marrow-derived vMSCs secrete an excessive amount of HGF, which is associated with angiogenesis, which has a paracrine effect that promotes the angiogenic sprouting of HUVECs.

### Example 7. Comparison of vMSCs and EPCs

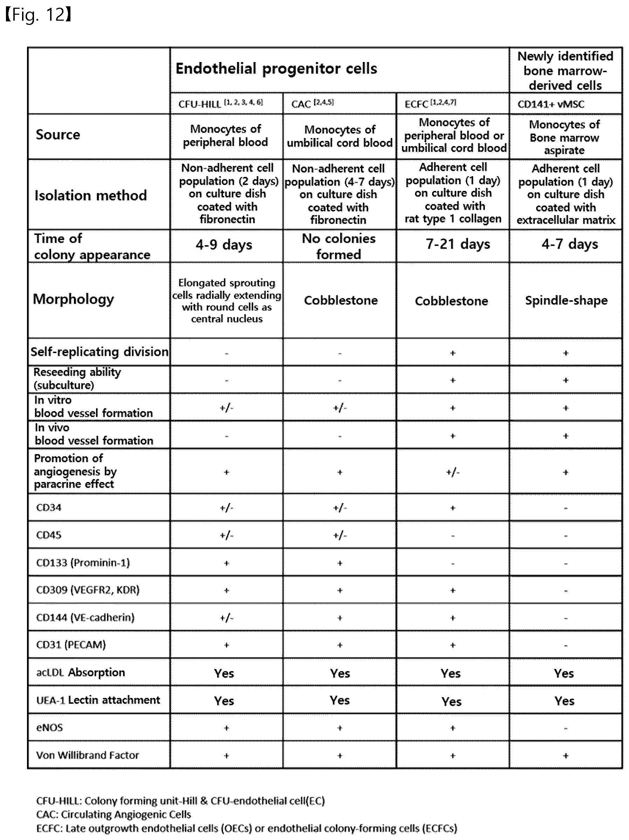

As a result of comparing the bone marrow-derived CD141⁺vMSCs of the present disclosure with peripheral blood or umbilical cord blood-derived EPCs confirmed through the results, the CD141⁺vMSCs of the present disclosure and EPCs had functionally similar characteristics, but the culture conditions, the cell morphology, and the expression of cell surface markers were completely different from each other, and in particular, CD309 and CD31, which were expressed in all types of EPCs, were hardly expressed in the CD141⁺vMSCs of the present disclosure. In addition, unlike EPCs of which sprouting was promoted by VEGF, the CD141⁺vMSCs of the present disclosure are not sprouted by VEGF and did not express VEGF receptors. In addition, the CD141⁺vMSCs had multipotency that has not been reported in conventional EPCs. Therefore, it can be seen that the CD141⁺vMSC cells of the present disclosure are a type of cell that is functionally similar to, but completely different from the commonly known EPCs (FIG. 12).

Through Examples above, the novel stem cells CD141⁺vMSCs identified in the present disclosure did not express CD31, CD309, and CD34 unlike EPCs, not expressed eNOS and VE-cadherin unlike vascular endothelial cells, expressed CD141 unlike mesenchymal stem cells, overexpressed HGF and its receptor cMET compared to mesenchymal stem cells, and had multipotency and angiogenesis-promoting effects. Therefore, the CD141⁺vMSCs were named CD141⁺Vasculogenic Multipotent Stem Cells and deposited with the Korea Cell Line Research Foundation (KCLRF) and assigned the accession number KCLRFBP00524.

### Example 8. Combination of bone marrow-derived vMSCs and BM-MSCs [00222] 8-1. Investigation of combination to maximize blood vessel regeneration ability in normal environment

To maximize the blood vessel regeneration ability of CD141⁺vMSCs, a Matrigel tube formation assay was performed to identify an optimal mixing ratio of two types of cells while adding BM-MSCs. Specifically, Matrigel was dispensed onto a µ-slide at 10 µl per well and gelled at 37°C for 30 minutes, and CD141⁺vMSCs and BM-MSCs were mixed in α-MEM (MEMα + 0.2%hPL) containing 0.2% hPL at a cell number ratio of 1:0 (6,000+0), 9:1 (5,400+600), 2:1 (4,000+2,000), 1:1 (3,000+3,000), 1:2 (2,000+4,000), 1:9 (600+5,400), and 0:1 (0+6,000), dispensed onto Matrigel, and cultured for 16 hours. Images of each group were obtained using a microscope (Nikon) and the total length, the number of meshes, the number of master segments, the total master segment length, and the number of isolated segments were used as evaluation indices using an Angiogenesis analyzer of Image J software.

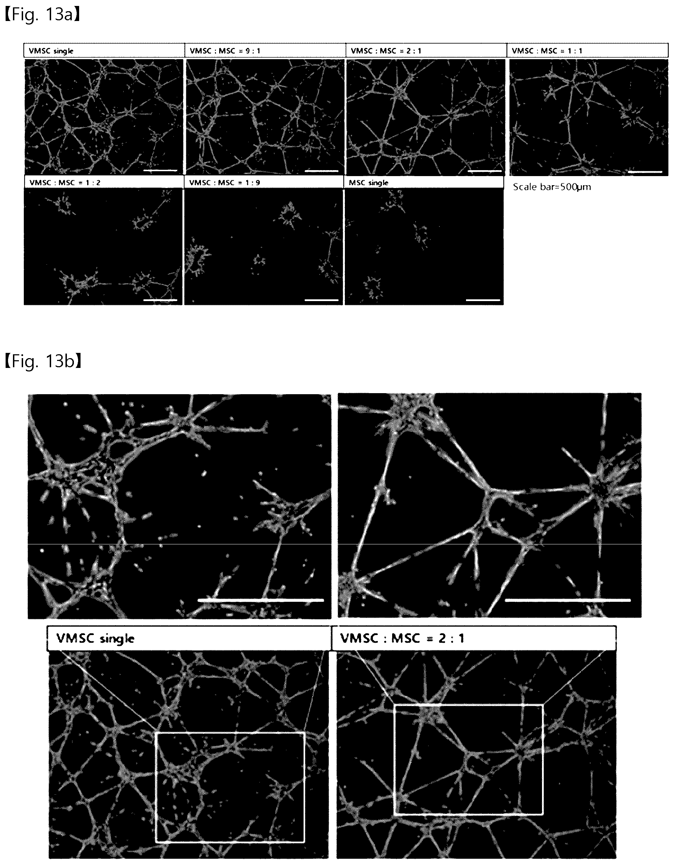

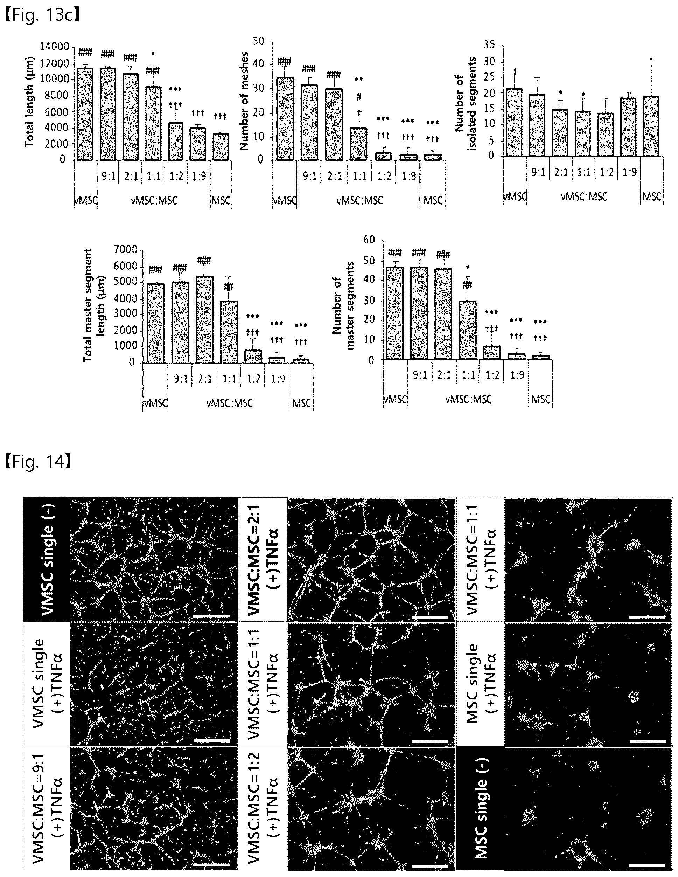

As the microscopic observation result, it was shown that there were differences in the tube formation depending on a composition ratio of cells. Specifically, BM-MSCs did not form tubes alone, and showed a tendency to aggregate together as the ratio of BM-MSCs increased compared to CD141⁺vMSCs (FIG. 13A). In addition, in a normal environment, CD141⁺vMSC cells alone showed excellent tube formation ability in vitro, but a loose structure was observed, but when BM-MSCs were added, tighter and more mature tubes were formed (FIG. 13B). As the results of analyzing the evaluation indices, it was shown that there was no significant difference in the total length, the number of meshes, the number of master segments, and the total master segment length, but the number of isolated segments, which was an indicator of immature blood vessels and represented segments that were not connected to anything, was the lowest in 2:1 among the three groups with no difference in the remaining indices (FIG. 13C).

That is, it was confirmed that although CD141⁺vMSCs alone had excellent in vitro angiogenic ability in a normal environment, CD141⁺vMSCs may form tighter and more mature tubes when mixed with BM-MSCs at an appropriate ratio.

### 8-2. Investigation of combination to maximize blood vessel regeneration ability in inflammatory environment

Considering that a stem cell therapeutic agent will be administered in an inflammatory environment, a Matrigel tube formation assay was performed in an inflammatory environment. To simulate the inflammatory environment, a medium containing 500 pg/mL of TNF-α was used. CD141⁺vMSCs and BM-MSCs were mixed in MEMα+0.2% hPL supplemented with 500 pg/mL of TNF-α at ratios of 1:0 (6,000+0), 9:1 (5,400+600), 2:1 (4,000+2,000), 1:1 (3,000+3,000), 1:2 (2,000+4,000), 1:9 (600+5,400), and 0:1 (0+6,000), suspended, and dispensed, respectively. In addition, for comparison with the normal environment, a group in which CD141⁺vMSCs and BM-MSCs were suspended in MEMα+0.2% hPL without 500 pg/mL of TNF-α was added, and a total of 9 groups were compared.

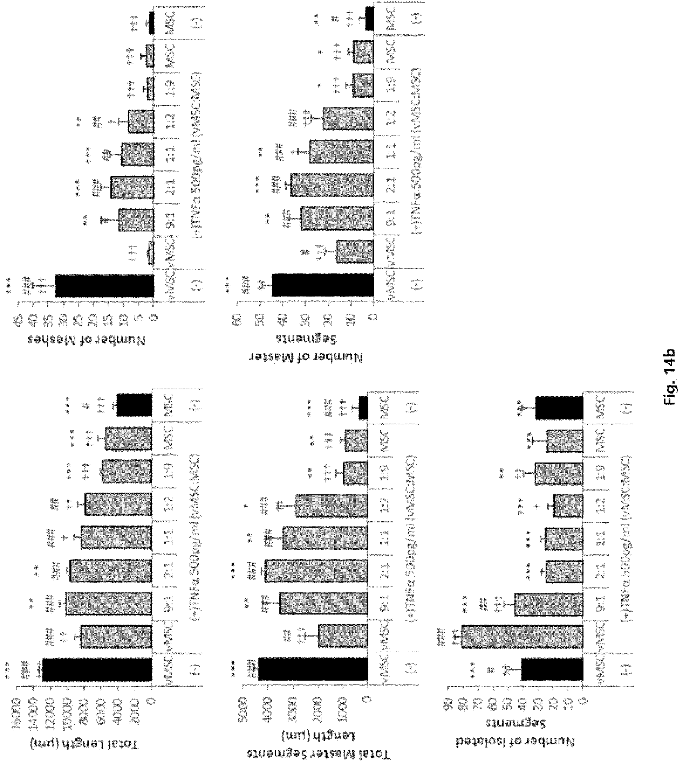

As the microscopic observation result, it was shown that there were differences in the tube formation depending on a composition ratio of cells. Specifically, CD141⁺vMSCs alone were able to form tubes well in a normal environment, but the tube formation ability decreased in an inflammatory environment. However, when BM-MSCs were added, the tube structure was found to be well formed, and as in the normal environment, an aggregation phenomenon was observed when the ratio of BM-MSCs increased (FIG. 14A). In addition, in the inflammatory environment, when CD141⁺vMSC cells were present alone, the total length was reduced, and in particular, the production of master segments and meshes, which were indicators of mature blood vessels, was most drastically reduced, while the number of isolated segments, which was an indicator of immature blood vessels, was drastically increased (FIG. 14B). On the other hand, when CD141⁺vMSC and BM-MSC cells were mixed in a 2:1 ratio, the length of the blood vessel itself and mesh production were maintained at a high level even in the inflammatory environment, and in particular, the number and length of the master segments were maintained at a high level similar to those of the CD141⁺vMSC-alone group in the normal environment, and the isolated segment length was observed at a lower level than that of the CD141⁺vMSC-alone group in the normal environment (FIG. 14B).

That is, it was confirmed that it was advantageous to use a combination of CD141⁺vMSCs and BM-MSCs for tight blood vessel formation in the inflammatory environment.

### Example 9. Adipose-derived vMSCs

### 9-1. Isolation of adipose tissue-derived vMSCs

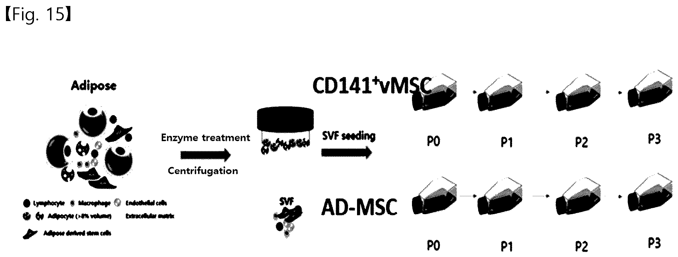

Stem cells of the mesodermal tissue of adipose tissue may produce bone, cartilage, muscle, and adipose tissue, and adipose tissue-derived stem cells, adipose-derived stem cells (ASCs), had the characteristics (cell shapes, expression markers, secreted cytokines, etc.) of bone marrow mesenchymal stem cells, had a larger initial tissue yield than bone marrow, and had an advantage of enabling cell transplantation at a faster passage, and thus have been currently used as a bone marrow substitute. Therefore, the adipose-derived vMSCs were isolated similarly to the method in Example 1 above. Specifically, adipose tissue (about 1.5 g) was obtained from a patient, chopped with scissors, isolated into single cells using collagenase I, and centrifuged to obtain stromal vascular fractions (SVFs). The SVFs were cultured in a FBS (fetal bovine serum)-deficient EGM2 (LONZA) + 2% PL (Platelet lysate, BioScience GmbH) medium on a culture dish coated with Humatein (Rokit healthcare) to obtain adipose-derived CD141⁺vMSCs (FIG. 15). To compare this, single cells isolated from adipose were cultured in a HUMA medium (Rokit healthcare) to obtain adipose-derived MSCs (AD-MSCs).

### 9-2. Cell morphology analysis of adipose-derived vMSCs

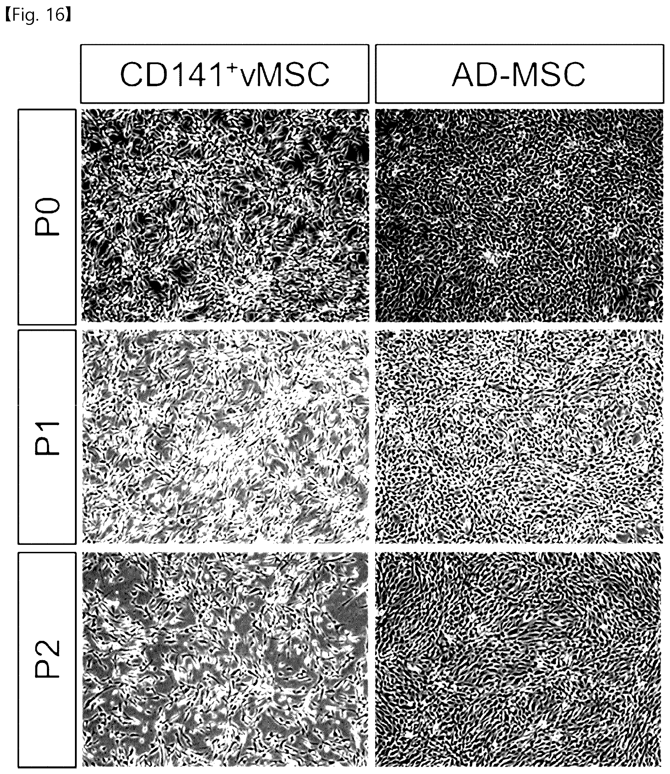

As a result of observing the cell shapes of the adipose-derived CD141⁺vMSCs and AD-MSCs obtained in Example 9-1 above by passage, the two types of cells had spindle shapes, which were a typical MSC cell shape, but CD141⁺vMSCs were found to have a longer and more pointed shape (FIG. 16).

### 9-3. Cell expansion ability analysis of adipose-derived vMSCs

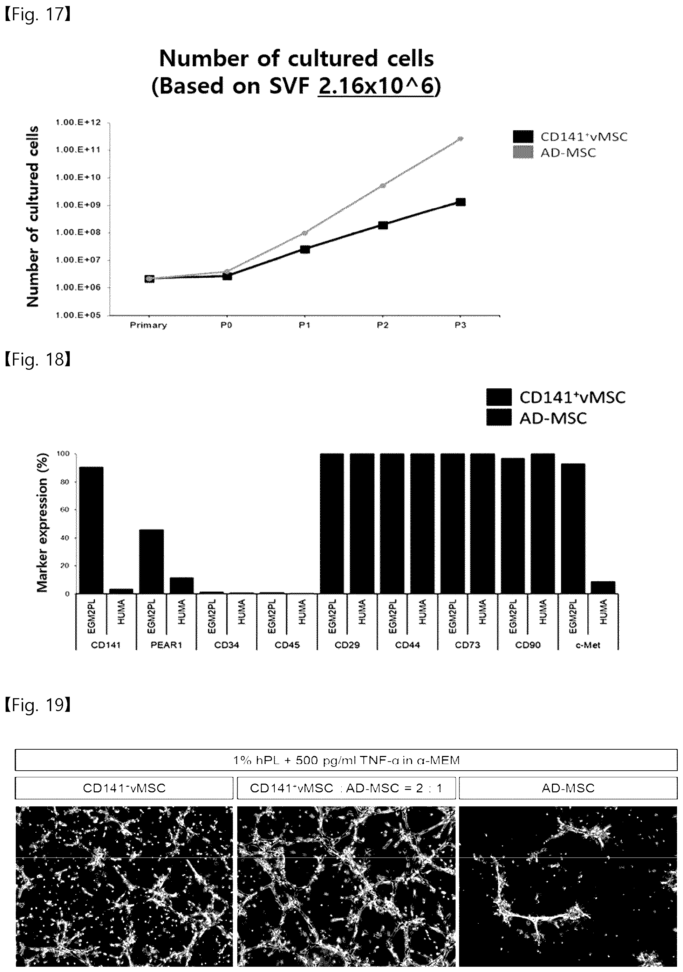

The cumulative cell numbers that could be obtained by dispensing adipose-derived CD141⁺vMSCs and AD-MSCs from the primary of the cell initial stage to Passage 3 (both cells starting from 2.16 × 10⁶ cells) were confirmed. As a result, at least 10⁹ adipose-derived CD141⁺vMSCs could be obtained in Passage 3 (FIG. 17).

### 9-4. Analysis of cell surface markers of adipose-derived vMSCs

As a result of analyzing the cell surface markers of adipose-derived CD141⁺vMSCs and AD-MSCs by flow cytometry, it was showed that both types of cells exhibited the characteristics of CD34-, CD45-, CD29+, CD44+, CD73+, and CD90+, the expression of CD141 and PEAR1 was high in CD141⁺vMSCs (EGM2-FBS+2%hPL) and low in AD-MSCs (HUMA) (FIG. 18), and thus it was confirmed that these two markers could be used as important markers to distinguish adipose-derived CD141⁺vMSCs from AD-MSCs.

### 9-5. Analysis of angiogenic ability of adipose-derived vMSCs

To confirm the angiogenic ability of adipose-derived CD141⁺vMSCs in an inflammatory environment to simulate a post-transplantation environment, adipose-derived CD141⁺vMSCs and AD-MSCs were dispensed singly (6,000 cells) or mixed in a 2:1 ratio (vMSC 4,000 cells + AD-MSC 2,000 cells) on Matrigel together with 1% hPL + 500 pg/ml TNF-α, and the blood vessel shapes that appeared after 24 hours were compared. As a result, in the case of AD-MSCs alone, the angiogenic ability was low and the cells were aggregated together, whereas in the case of adipose-derived CD141⁺vMSCs alone or when CD141⁺vMSCs and AD-MSCs were mixed in a 2:1 ratio, the angiogenic ability increased compared to when AD-MSCs were cultured alone. In particular, it was shown that the angiogenic ability was the best when CD141⁺vMSCs and AD-MSCs were mixed in a 2:1 ratio (FIG. 19).

### Example 10. Establishment of critical limb ischemia animal model

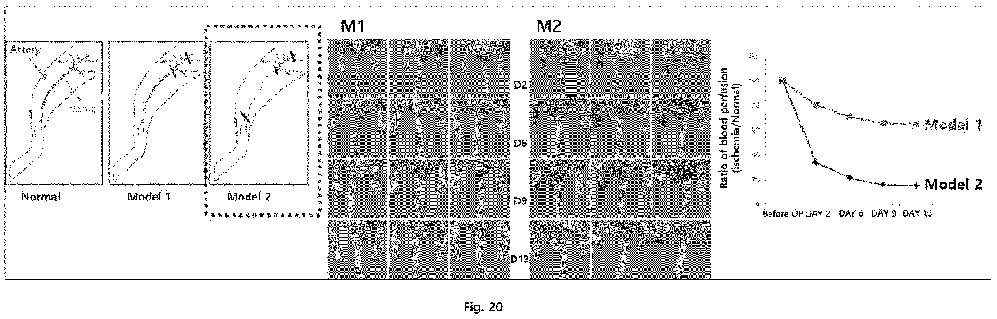

To confirm the efficacy of a combination of bone marrow-derived CD141⁺vMSCs and BM-MSCs as a composite stem cell therapeutic agent in critical limb ischemia, a critical limb ischemia animal model was fabricated. Model 1 of FIG. 20 was a model in which the upper parts of the common femoral artery and the superficial femoral artery were ligated using silk, and since a blood flow was recovered over time in most mice, it was determined to be unsuitable as a model for observing blood vessel formation and evaluating effectiveness. Therefore, Model 2 (critical limb ischemia, CLI) was fabricated by ligating three places, i.e. both sides of the common femoral artery and the superficial femoral artery, and then removing the superficial femoral artery, which showed symptoms of severe necrosis and tissue loss and a decreased blood flow over time in most mice (FIG. 20).

### Example 11. Confirmation of effect of composite stem cells on critical limb ischemia animal model

### 11-1. Evaluation of limb salvage rate

The efficacy of CD141⁺vMSC + BM-MSC as a composite stem cell therapeutic agent was evaluated in the critical limb ischemia animal model (Model 2) fabricated in Example 10 above, and the effect thereof was observed for up to 24 weeks. Depending on an administered substance, a control group (G0) and a CD141⁺vMSC + BM-MSC (cell number 2:1) administered group (G1) were set, and intramuscularly administered to an ischemia site (vessel removal site) once at a dose of 1.2 × 10⁵ cells using an insulin syringe immediately after the fabrication of the critical limb ischemia model.

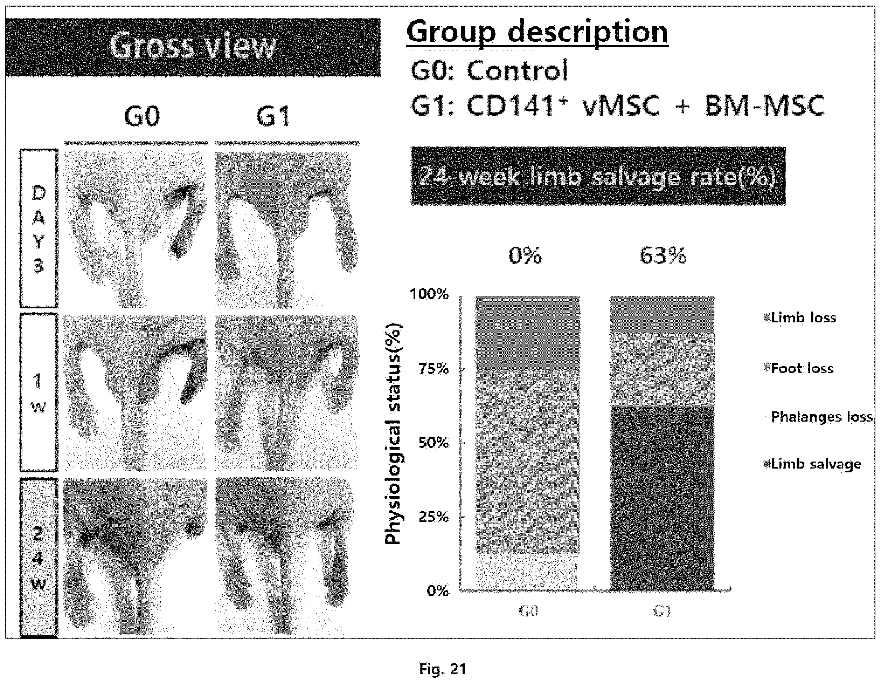

As a result, as a result of observing the final limb condition at 24 weeks after the model was fabricated, the limb salvage rate in the control group was 0%, and most subjects showed a limb/lower limb loss, but in the G1 group, the limb salvage was approximately 63% or more (FIG. 21).

### 11-2. Evaluation of ischemic necrosis grade

After administration to the critical limb ischemia mouse model as in Example 11-1, the severity of ischemic necrosis in the ischemic area was divided into 7 stages and graded and evaluated.

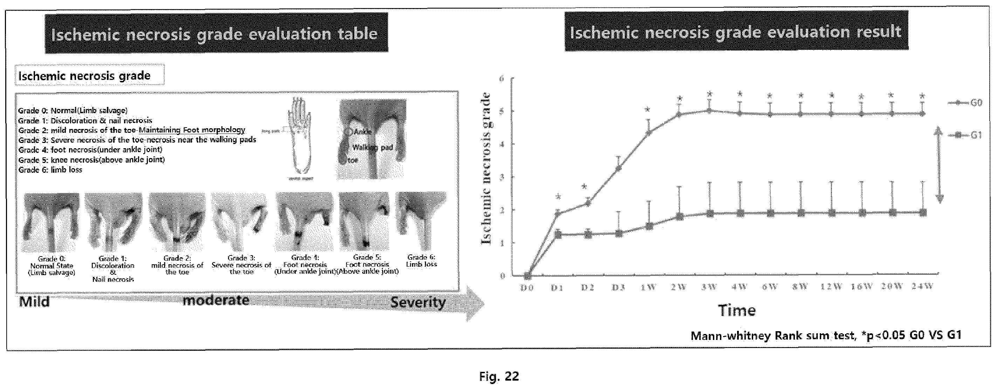

As a result, after the model was fabricated, a statistically significant difference was observed in the degree of ischemic necrosis in the G1 group compared to the G0 group on days 1 and 2 after administration, and a significant difference began to appear on week 1 and the statistically significant difference continued until week 24 (FIG. 22) (week 1, G0: 4.3 ± 0.8 VS G1: 1.5 ± 0.8, p < 0.05; and week 24, G0: 4.9 ± 0.4 VS G1: 1.9 ± 0.9, p < 0.05).

### 11-3. Analysis of blood flow changes

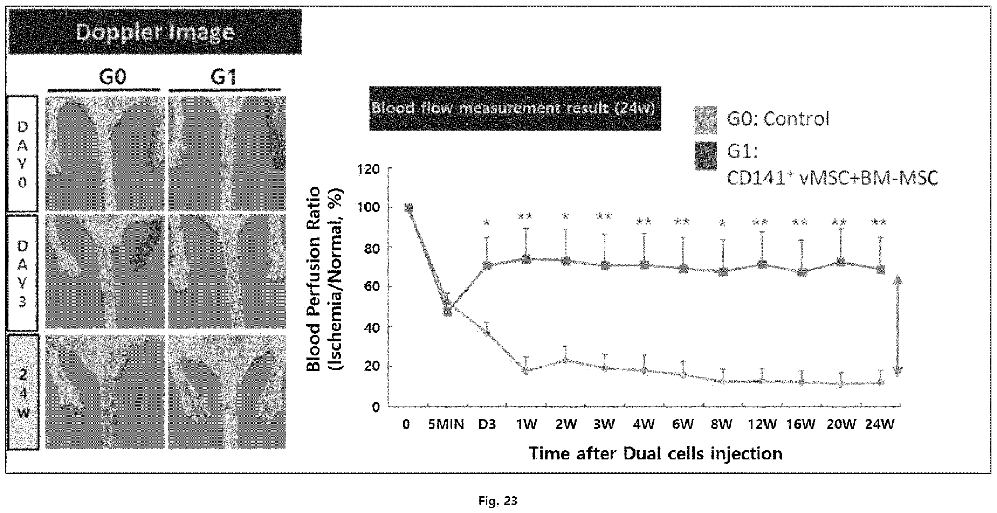

After administration to the critical limb ischemia mouse model as in Example 11-1, the blood flow between a contralateral side where ischemia did not occur and an ischemic side where ischemia occurred was compared and expressed as a ratio (%). As a result, after the fabrication of the critical limb ischemia mouse model, the blood flow was G0: 52.4 ± 4.9 and G1: 47.3 ± 1.4, and thus a rapid decrease in blood flow was confirmed in the two groups. However, from day 3, a significant improvement in blood flow began to appear in the G1 group compared to the G0 group, and such a statistically significant improvement was maintained up to week 24 (FIG. 23) (day 3, G0: 37.2 ± 4.9 VS G1: 70.8 ± 14.3, p < 0.05; and week 24, G0: 11.9 ± 6.3 VS G1: 69 ± 15.9, p < 0.01). Through this, it was confirmed that the combination of CD141⁺vMSC and BM-MSC had a valid effect in critical limb ischemia, and the blood flow recovered on day 3 to week 1 was maintained at a similar level for up to 24 weeks.

### 11-4. Determination of minimal effective dose

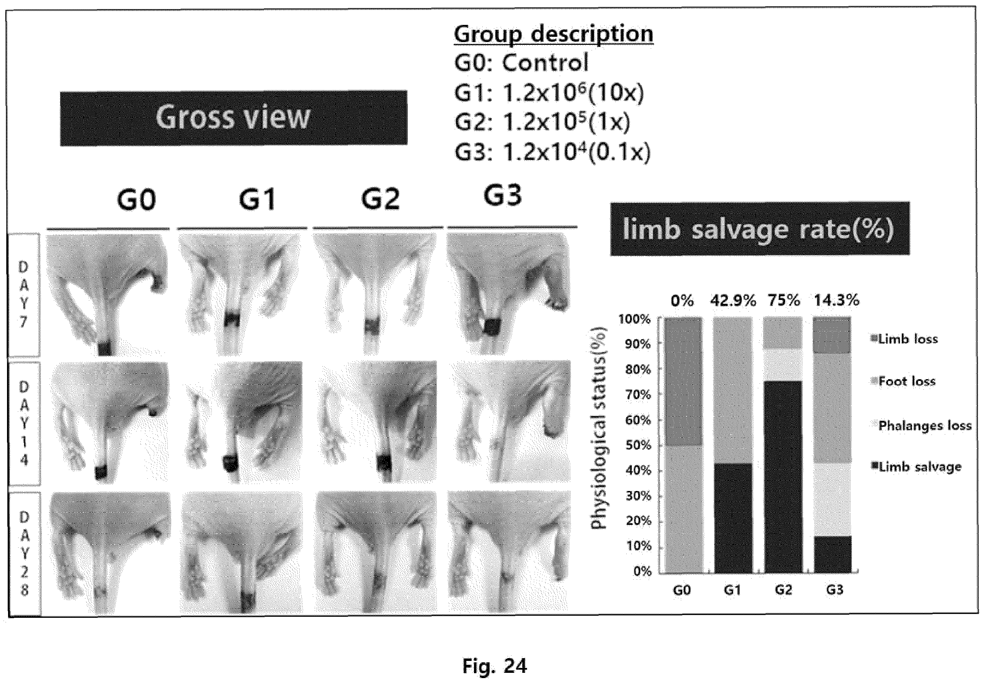

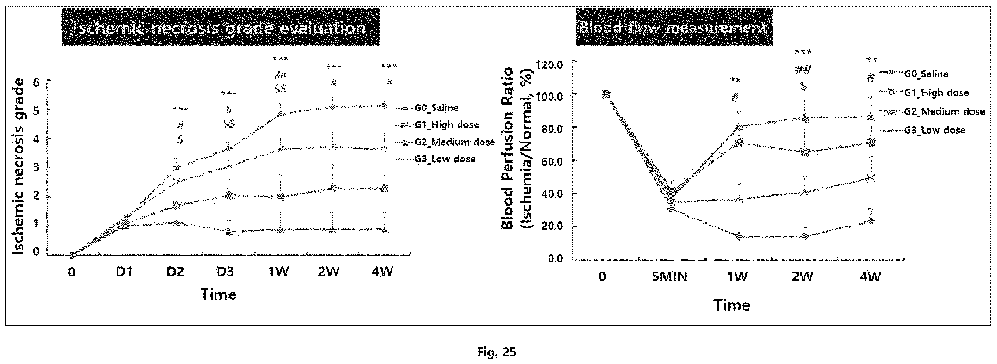

To establish an effective dose of the composite stem cell therapeutic agent according to a combination of CD141⁺vMSC and BM-MSC for critical limb ischemia and to determine a minimal effective dose, the composite stem cell therapeutic agent was divided into a control group (G0), a high-dose group (1.2 × 10⁶ cells/50 µL, 24,000/µL) (G1), a medium-dose group (1.2 × 10⁵ cells/50 µL, 2,400/µL) (G2), and a low-dose group (1.2 × 10⁴ cells) (G3), and administered to each group. Then, the final limb condition was observed on week 4 after model fabrication (D0), and as a result, the control group showed a limb salvage rate of 0%, and most of the limb/lower limb loss was observed, whereas the G1 and G2 groups showed high limb salvage rates of 42.9% and 75%, respectively (FIG. 24). In addition, in the G3 group, the limb salvage rate was 14.3%, and the phalange loss rate was 28.6%, which was relatively good (FIG. 24). In addition, as a result of evaluating the ischemic necrosis grade as an efficacy evaluation indicator and measuring a blood flow, statistically significant effects in the high-dose group and the medium-dose group were exhibited compared to the control group (FIG. 25). These effective effects were not dose-dependent, but were sufficiently effective at a high dose. Therefore, the effective dose was confirmed at high and middle doses, and then the minimal effective dose was determined as 1.2 × 10⁵ cells.

### Example 12. Comparison of effect of composite stem cell administration compared to single stem cell administration

### 12-1. Evaluation of limb salvage rate

To determine whether the administration of the composite stem cell of CD141⁺vMSCs and BM-MSCs was superior to the stem cell administration of CD141⁺vMSCs and BM-MSCs alone in a critical limb ischemia mouse model, a control group (G0), a CD141+vMSC+BM-MSC group (G1), a CD141+vMSC alone group (G2), and a BM-MSC group (G3) were set, and each was administered at a dose of 1.2 × 10⁵ Cells.

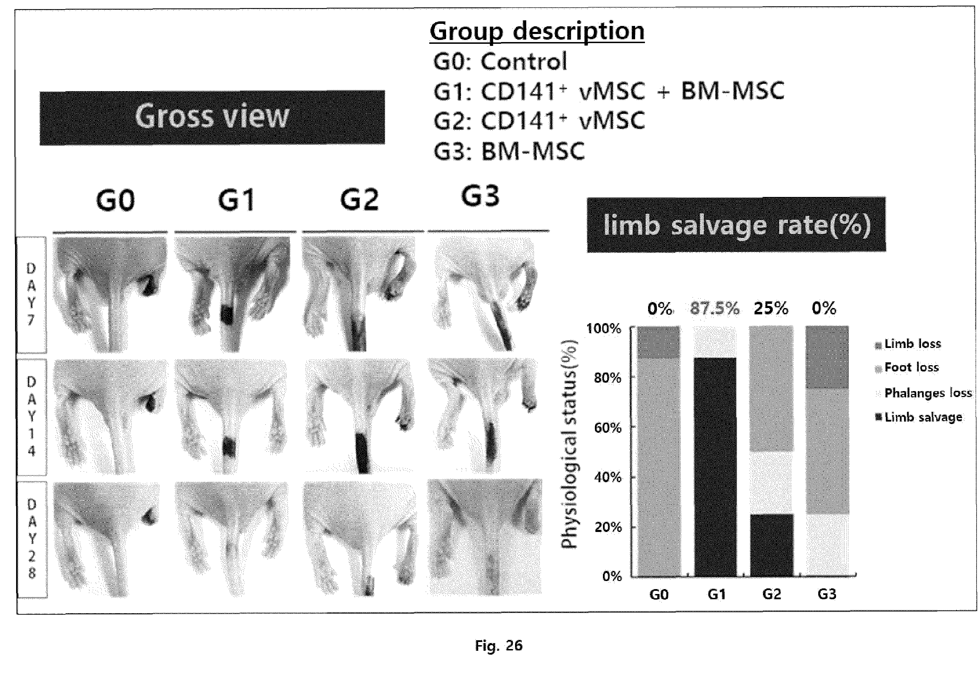

As a result of observing the final limb condition 4 weeks (DAY28) after model fabrication, the control group showed a limb salvage rate of 0% and most subjects showed limb/lower limb loss, whereas the G1 group showed limb salvage of approximately 87.5% or more, the G2 group showed relatively good limb salvage rate of 25% and phalange loss of 25%, and the G3 group showed a limb salvage rate of 0% and phalange loss of 25% (FIG. 26).

### 12-2. Evaluation of ischemic necrosis grade

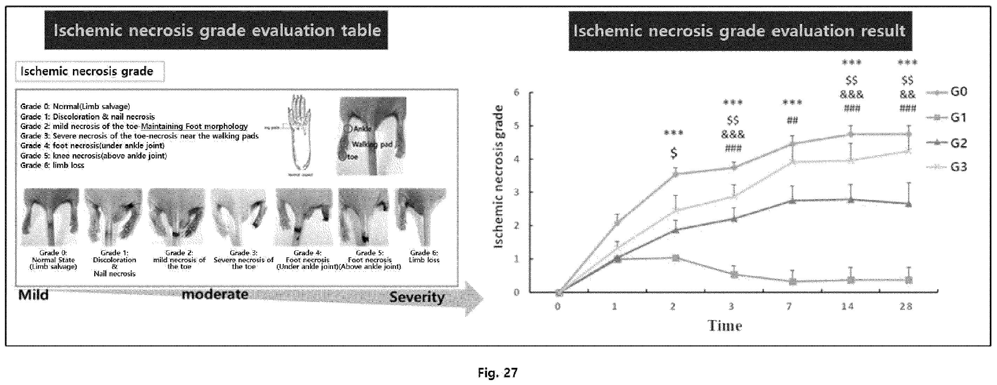

As a result of evaluating an ischemic area by dividing and grading the ischemic necrosis into 7 stages accordign to the severity thereof in each group of Example 12-1 above, it was confirmed that there was no statistically significant difference between each group at model fabrication (time 0) and 1 day after administration (time 1), but from day 2, the G1 group began to show a statistically significant difference compared to the G0 group, and the significant difference was maintained until day 28 (FIG. 27) (2 days after administration, G0: 3.5 ± 0.2 VS G1: 1.0 ± 0, P < 0.001; and 28 days, G0: 4.8 ± 0.3 VS G1: 0.4 ± 0.4, P < 0.001). In addition, even in the comparison of combined/single administration, the G1 group showed a statistically significant result compared to the G3 group from day 3 to day 28 (day 3, G1 VS G3: 2.9 ± 0.4, P < 0.001; and day 28, G1 VS G3: 4.3 ± 0.5, P < 0.001), and in the comparison with the G2 group, a statistically significant difference was confirmed on days 3, 14, and 28 (FIG. 27) (day 3, G1 VS EPC G2: 2.2 ± 0.3, P < 0.001; and day 28, G1 VS G2: 2.7 ± 0.6, P < 0.01).

### 12-3. Analysis of blood flow changes

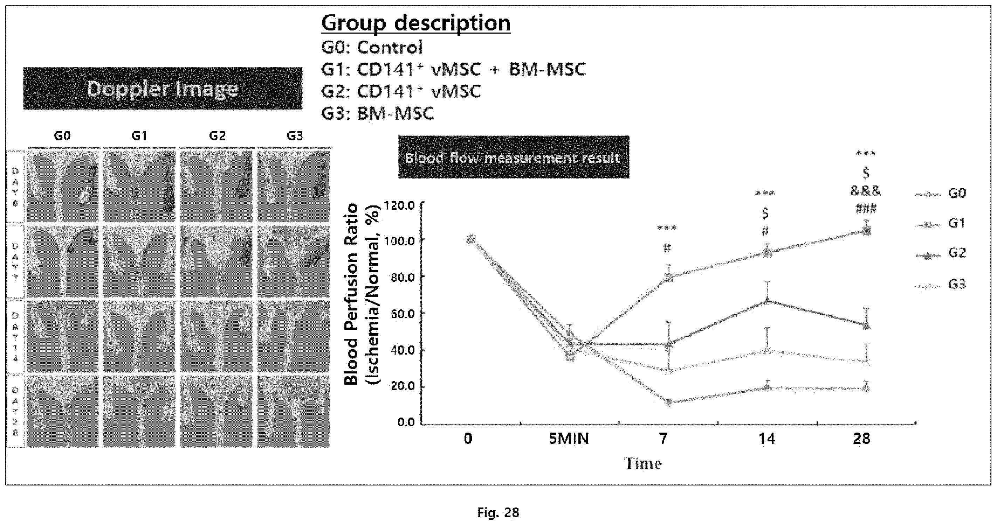

In each group of Example 12-1 above, the blood flow between the contralateral side where ischemia did not occur and the ischemic side occurred was compared and expressed as a ratio (%), and as a result, it was confirmed that the blood flow was drastically reduced after a CLI model was fabricated. On the other hand, it was confirmed that a statistically significant improvement in blood flow began to appear in the G1 group compared to the G0 group from day 7, and such a statistically significant improvement was maintained until day 28 (FIG. 28) (day 7, G1: 79.4 ± 6.8 VS G0: 11.8 ± 1.9, p < 0.001; and day 28, G1: 104.6 ± 5.5 VS G0: 19.4 ± 4.0, p < 0.001). In addition, even in the comparison of combined/single administration, the G1 group showed a statistically significant improvement compared to the G3 group from day 3 to day 28, and in the comparison with the G2 group, a statistically significant difference was confirmed on days 3, 14, and 28 (FIG. 28) (day 7, G1: 79.4 ± 6.8 VS G3: 28.8 ± 11.0, p < 0.05; day 28, G1: 104.6 ± 5.5 VS G3: 33.6 ± 10.1, p < 0.001, G2: 53.9 ± 9.3, p < 0.001).

Through this, it was confirmed that the administration of the composite stem cells according to a combination with CD141⁺vMSCs and BM-MSCs had a superior therapeutic effect in critical limb ischemia compared to the administration of each cell alone.

### Example 13. In vivo angiogenic analysis of composite stem cells

### 13-1. Confirmation of angiogenesis (visually)

CD141⁺vMSCs and BM-MSCs were combined (CD141+vMSCs + BM-MSCs) and administered to an ischemic site, which was a vascular removal site in a critical limb ischemia mouse model, and then angiogenesis in vivo was observed at 4 weeks.

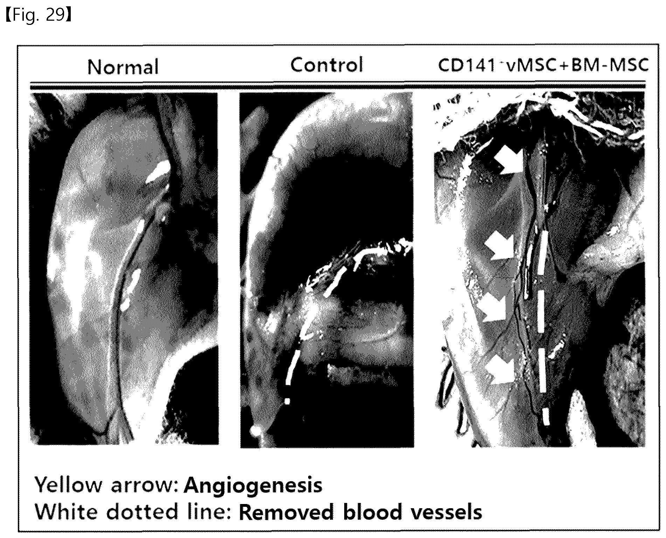

As a result, in normal group (Normal) mice that were not induced with critical limb ischemia, the femoral artery was clearly present and the muscle tissue was also well preserved, whereas in control group (Control) mice that were induced with critical limb ischemia, severe inflammation and damage to the surrounding tissue were observed near the removed blood vessel. In contrast, it was confirmed that in the group administered with a combination of CD141⁺vMSC and BM-MSC (CD141⁺vMSC+BM-MSC), new blood vessels were formed and observed near the removed blood vessels (yellow arrows), and the surrounding tissues were also very well preserved (FIG. 29).

### 13-2. Confirmation of angiogenesis (immunofluorescence staining)

In mice in each group of Example 13-1 above, CD31+ blood vessel staining of the entire muscle was performed using 3D immunofluorescence staining to observe blood vessel density.

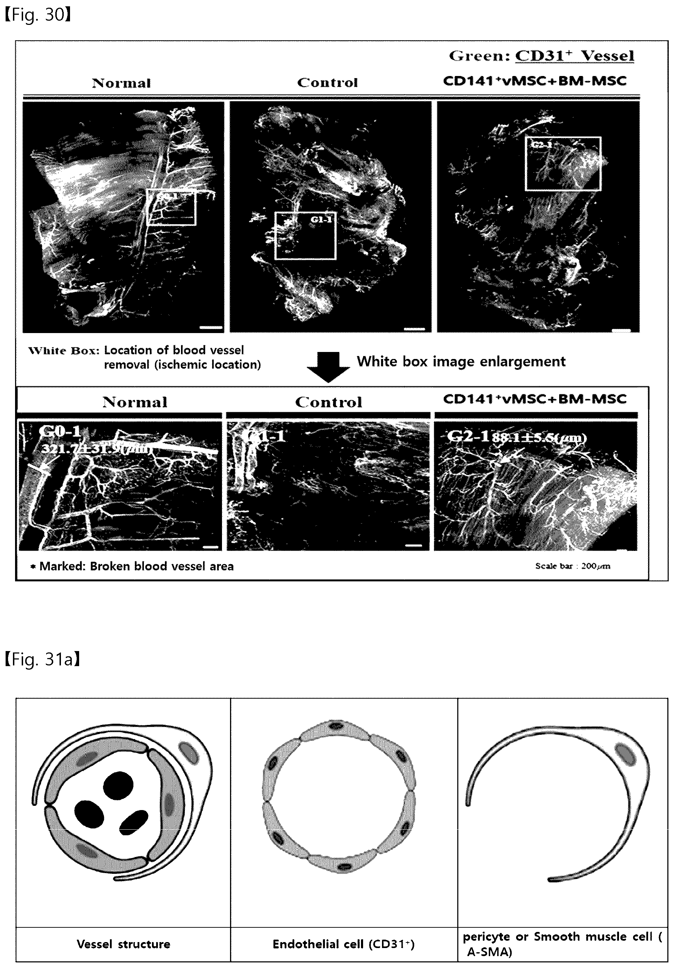

As a result, it was shown that in the case of the normal group (Normal) mice, small arteries were extended based on the femoral artery, while in the control group (Control) mice, the arteries were broken (* marked) and the vascular density of the ischemic lesion was very low. On the other hand, in the group administered with the combination of CD141⁺vMSC and BM-MSC (CD141⁺vMSC+BM-MSC), it was confirmed that small arterioles were formed at a high density (FIG. 30). Even in the result of enlarging and confirming the vascular staining images, a thick artery (femoral artery) with an inner diameter of 321.7 ± 31.9 µM was observed in the normal group, and a small artery with an inner diameter of 88.1 ± 5.5 µM was observed to extend to the vessel removal site in the CD141⁺vMSC+BM-MSC group, whereas in the control group, broken blood vessels were observed (*), and the blood vessel density around the broken blood vessels was very low, and only some thin blood vessels were observed (FIG. 30).

### 13-3. Identification of angiogenic markers

Since a structure of blood vessel consisted of endothelial cells and pericytes (smooth muscle cells) surrounding the endothelial cells (FIG. 31A), after CD141⁺vMSC + BM-MSC was administered into the muscle of the ischemic lesion of the critical limb ischemia mouse model (2:1), immunofluorescence staining and immunohistological staining were performed using anti-human-CD31 antibody for CD31 as an endothelial cell marker, and anti-human-A-SMA antibody for A-SMA (alpha-smooth muscle actin) as a pericyte marker.

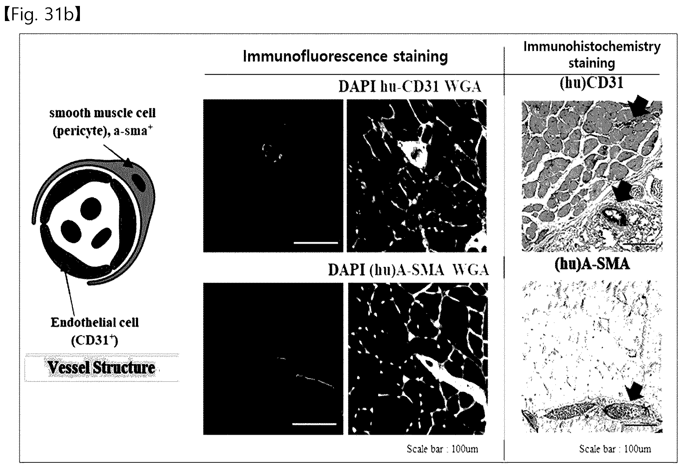

As a result, it was shown that Human-CD31 and Human A-SMA formed blood vessels in the muscle, which was the administration site of CD141⁺vMSC + BM-MSC (FIG. 31B). Therefore, it was confirmed that CD141⁺vMSC + BM-MSC may form blood vessels in vivo.

### 13-4. Classification according to vessel diameter

To confirm the angiogenic ability according to the combined administration of CD141⁺vMSC and BM-MSC, CD141⁺vMSC and BM-MSC were combined and administered to the muscle of the ischemic lesion of the critical limb ischemia mouse model as in Example 13-3 above, and then the muscle of the ischemic lesion was stained with immunofluorescence staining using an antibody against TAGLN (Transgelin), a marker of smooth muscle cells, which were cells constituting blood vessels, and TAGLN+ blood vessels were analyzed.

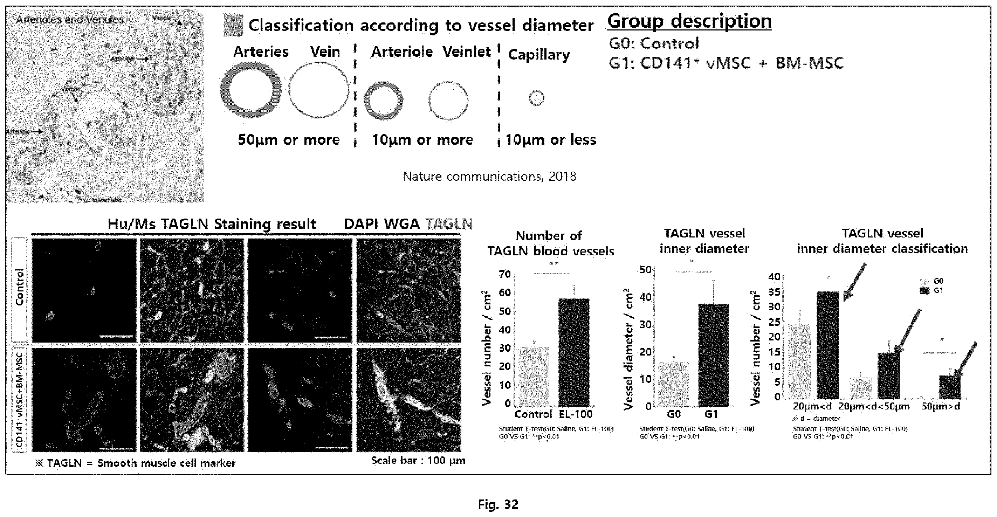

As a result, the group administered with CD141⁺vMSC + BM-MSC in the ischemic lesion muscle showed a statistically significant increase in the number of TAGLN+ blood vessels compared to the control group (Control) (FIG. 32) (Control: 31.6 ± 3.0 VS composite stem cells: 57 ± 6.8, p < 0.01). In addition, even in the result of measuring the vessel inner diameter size, it was shown that the vessel inner diameter size of the CD141⁺vMSC + BM-MSC administration group was significantly larger than that of the control group (FIG. 32) (Control: 15.9 ± 1.9 µM VS composite stem cells: 36.8 ± 8.4 µm, p < 0.5). Based on the results, the blood vessels were classified by the size of the vessel diameter and the number of blood vessels was measured. As a result, it was confirmed that when the diameters were 20 µm or less and 20 µm to 50 µm or less, the number of blood vessels in the control group and the CD141⁺vMSC + BM-MSC administration group was not statistically significant, but the number of blood vessels was greater in the CD141⁺vMSC + BM-MSC administration group. In addition, in cases of 50 µm or more, the number of blood vessels in the CD141⁺vMSC + BM-MSC administration group was significantly greater than that in the control group (FIG. 32) (Control: 0 VS CD141⁺vMSC + BM-MSC:7 ± 2, p < 0.05).

As a result, it was confirmed that combined administration of CD141⁺vMSCs and BM-MSCs could increase a blood flow in the ischemic area by not only increasing the number of blood vessels but also forming blood vessels with a large inner diameter size of 50 µm or more.

### Example 14. Confirmation of in vivo safety of composite stem cells

To confirm the safety after combined/co-administration of CD141⁺vMSCs and BM-MSCs in normal BALB/c Nude mice, the in vivo distribution was confirmed by immunohistochemistry (IHC) to confirm the formation of CD31+ blood vessels and Alpha-SMA+ blood vessels. In addition, in order to confirm an engraftment pattern, the numbers of CD31+ blood vessels and Alpha-SMA+ blood vessels were confirmed at 1, 4, 13, and 26 weeks. In addition, a body distribution test was performed using qPCR in blood and tissues/organs at 6 hours, 1 day, 1 week, 4 weeks, 13 weeks, and 26 weeks after single administration. In addition, toxicity was confirmed for 26 weeks after single administration of a dose of 1.2 × 10⁵ cells, 6 × 10⁵ cells, or 1.2 × 10⁶ cells intramuscularly. In addition, tumor formation and potential were observed for up to 26 weeks after a single subcutaneous administration at a dose of 1 × 10⁷ cells and a single intramuscular administration at a dose of 2.4 × 10⁶ cells.

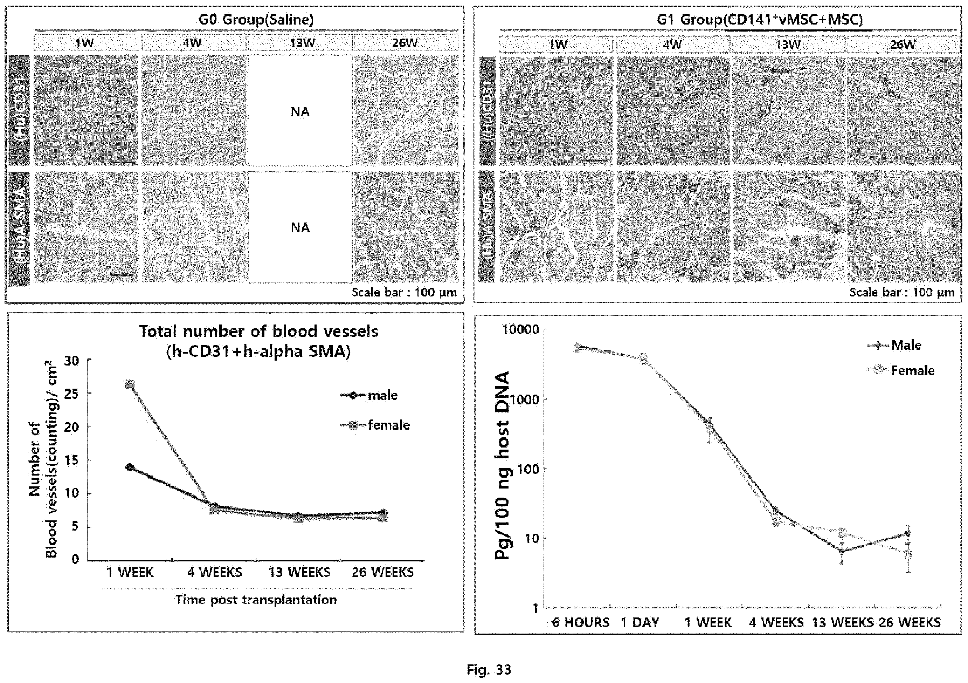

As IHC analysis results, it was shown that combined/co-administration of CD141⁺vMSCs and BM-MSCs significantly increased the formation of CD31+ blood vessels and Alpha-SMA+ blood vessels compared to the control group (G0) (FIG. 33). In addition, as a result of confirming the number of blood vessels over time after administration, it was found that the number of blood vessels was the highest on week 1, decreased until week 4, and then maintained at a similar level until week 26 (total number of blood vessels in FIG. 33). As a result of the body distribution test using qPCR, composite stem cells were detected only in the muscles at the administration site at all observation time points, and were not detected in other organs and tissues, and the administered cells were detected in all subjects until week 4, but were also detected in most subjects at weeks 13 and 26 (Pg/100 ng host DNA in FIG. 33). Through this, it can be seen that the combined/co-administration of CD141⁺vMSC and BM-MSC remained and engrafted only in the muscle of the administration site for a long time, and differentiated into a vascular form, and thus the effect of treating critical limb ischemia was maintained. In addition, no toxic response was observed at all doses for 26 weeks (data not shown), and thus a no-toxic dose was set to approximately 1.2 × 10⁶ cells. In addition, as a result of observing tumor formation and potential, it was confirmed that no tumors were formed in association with the test substance, so that there was no tumor formation and potential. Through this, it was confirmed that there was no toxicity or tumor formation by the combined/co-administration of CD141⁺vMSC and BM-MSC, and thus there were no safety issues when using the combined CD141⁺vMSC and BM-MSC as a therapeutic agent for critical limb ischemia.

## Claims

1. Stem cells expressing a CD141 (thrombomodulin TM) cell surface antigen.

2. The stem cells of claim 1, wherein the stem cells are vasculogenic multipotent stem cells (vMSCs).

3. The stem cells of claim 2, wherein the stem cells are deposited under accession number KCLRF-BP-00524.

4. The stem cells of claim 2, wherein the stem cells are umbilical cord blood-derived, umbilical cord-derived, adipose-derived or bone marrow-derived.

5. The stem cells of claim 2, wherein the stem cells have blood vessel regeneration ability or angiogenic ability.

6. The stem cells of claim 2, wherein the stem cells have multipotency.

7. The stem cells of claim 2, wherein the stem cells overexpress a hepatocyte growth factor (HGF) and a receptor thereof compared to other stem cells.

8. The stem cells of claim 2, wherein a population doubling time (PDT) is 0.5 to 1.5 days.

9. The stem cells of claim 2, wherein the stem cells promote angiogenesis by a basic fibroblast growth factor (bFGF) or HGF.

10. The stem cells of claim 2, wherein the stem cells do not express a CD31, CD309, CD34, eNOS, VE-cadherin, or VEGF receptor.

11. A cell therapeutic agent composition for blood vessel regeneration or angiogenesis, comprising the stem cells of claim 1 as an active ingredient.

12. The cell therapeutic agent composition for blood vessel regeneration or angiogenesis of claim 11, further comprising: bone marrow-derived mesenchymal stem cells or adipose-derived mesenchymal stem cells.

13. The cell therapeutic agent composition for blood vessel regeneration or angiogenesis of claim 12, wherein the bone marrow-derived mesenchymal stem cells or adipose-derived mesenchymal stem cells do not express a CD141 cell surface antigen.

14. The cell therapeutic agent composition for blood vessel regeneration or angiogenesis of claim 12, wherein the stem cells of claim 1 and the bone marrow-derived mesenchymal stem cells or adipose-derived mesenchymal stem cells are included in a ratio of 1:10 to 10:1 based on the cell number.

15. The cell therapeutic agent composition for blood vessel regeneration or angiogenesis of claim 11, further comprising: HGF or bFGF.

16. A graft material for blood vessel regeneration, comprising the stem cells of claim 1 or the cell therapeutic agent composition of claim 11 as an active ingredient.

17. A pharmaceutical composition for preventing or treating vascular disease or vascular dysfunction, comprising the stem cells of claim 1 or the cell therapeutic agent composition of claim 11 as an active ingredient.

18. The pharmaceutical composition for preventing or treating vascular disease or vascular dysfunction of claim 17, wherein the vascular disease is traumatic vascular injury, peripheral vascular disease, cardiovascular disease, cerebrovascular disease, or ischemic disease.

19. The pharmaceutical composition for preventing or treating vascular disease or vascular dysfunction of claim 18, wherein the ischemic disease is ischemic myocardial infarction, ischemic heart disease, ischemic vascular disease, ischemic enteritis, ischemic eye disease, ischemic glaucoma, ischemic renal failure, ischemic retinopathy, ischemic stroke, or ischemic lower extremity disease.

20. The pharmaceutical composition for preventing or treating vascular disease or vascular dysfunction of claim 17, wherein the vascular disease or vascular dysfunction is critical limb ischemia, diabetic angiogenic disorders, vascular dementia, diabetic organ damage, arteriosclerosis, angina, peripheral cardiovascular disease, hypertension, cerebral infarction, brain injury sequelae, heart failure, peripheral circulatory disorders, myocardial infarction, arterial occlusive disease, stroke, spinal cord injury, spinal nerve sequelae, degenerative disease, cerebral infarction sequelae, peripheral neuropathy, diabetic ulcer, presbyopia, degenerative hearing loss, brain surgery sequelae, ischemic stroke, or subarachnoid hemorrhage.

21. A pharmaceutical composition for preventing or treating brain neurological disease, comprising the stem cells of claim 1 or the cell therapeutic agent composition of claim 11 as an active ingredient.

22. The pharmaceutical composition for preventing or treating brain neurological disease of claim 21, wherein the brain neurological disease is a degenerative brain disease, a mental disorder, or a developmental disorder.

23. The pharmaceutical composition for preventing or treating brain neurological disease of claim 22, wherein the degenerative brain disease is cognitive impairment, Alzheimer's disease, dementia with Lewy bodies, frontotemporal dementia, Parkinson's disease, Creutzfeldt-Jakob disease (CJD), Huntington's disease, multiple sclerosis, or Guillain-Barre Syndrome (GBS).

24. The pharmaceutical composition for preventing or treating brain neurological disease of claim 22, wherein the mental disorder is bipolar disorder, autism, depression, hyperactivity, attention deficit, autism, post-traumatic stress disorder (PTSD), anxiety disorder, sleep disorder, panic disorder, intellectual disability, memory impairment, drug addiction, schizophrenia, obsessive-compulsive disorder, delusions of grandeur, personality disorder, alcoholism, or manic depression.

25. The pharmaceutical composition for preventing or treating brain neurological disease of claim 22, wherein the developmental disorder is epilepsy, cerebral palsy, developmental language disorder, disturbance of sense, learning disorder, attention deficit hyperactivity disorder (ADHD), autism spectrum disorder (ASD), intellectual disability, cognitive impairment, or impulse control disorders.

26. A method for preparing stem cells expressing a CD141 cell surface antigen, comprising culturing human bone marrow mononuclear cells (cryopreserved, BM-MNCs) or adipose-derived stroma-vascular fractions (SVFs) in a medium containing a human platelet lysate without containing serum.

27. A use of stem cells expressing a CD141 cell surface antigen, for use in blood vessel regeneration or angiogenesis.

28. A method for blood vessel regeneration or angiogenesis, comprising administering stem cells expressing a CD141 cell surface antigen to a subject.

29. A method for treating vascular disease or vascular dysfunction, comprising administering stem cells expressing a CD141 cell surface antigen to a subject suffering from vascular disease or vascular dysfunction.

30. A method for treating brain neurological disease, comprising administering stem cells expressing a CD141 cell surface antigen to a subject suffering from brain neurological disease.
